(19) Europäisches Patentamt / European Patent Office / Office européen des brevets

(11) **EP 2 860 250 B1**

(12) **EUROPEAN PATENT SPECIFICATION**

(45) Date of publication and mention
of the grant of the patent:
**19.12.2018 Bulletin 2018/51**

(51) Int Cl.:
*C12N 15/09* (2006.01)  *A61K 39/395* (2006.01)
*C07K 16/40* (2006.01)  *G01N 27/62* (2006.01)
*G01N 33/53* (2006.01)  *C12P 21/08* (2006.01)
*G01N 33/574* (2006.01)  *C07K 16/30* (2006.01)

(21) Application number: **13803567.0**

(22) Date of filing: **10.06.2013**

(86) International application number:
**PCT/JP2013/065975**

(87) International publication number:
**WO 2013/187371 (19.12.2013 Gazette 2013/51)**

(54) **METHOD FOR DETECTING CANCER, AND ANTIBODY CAPABLE OF RECOGNIZING PANCREAS-SPECIFIC RIBONUCLEASE 1**

VERFAHREN FÜR DEN NACHWEIS VON KREBS UND ANTIKÖRPER ZUR ERKENNUNG VON PANKREASSPEZIFISCHER RIBONUKLEASE 1

MÉTHODE POUR LA DÉTECTION DU CANCER, ET ANTICORPS APTE À RECONNAÎTRE LA RIBONUCLÉASE 1 SPÉCIFIQUE DU PANCRÉAS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.06.2012 JP 2012131970**
**13.12.2012 JP 2012272285**

(43) Date of publication of application:
**15.04.2015 Bulletin 2015/16**

(73) Proprietor: **Tosoh Corporation**
**Yamaguchi 746-8501 (JP)**

(72) Inventor: **NAKATA, Daisuke**
**Kanagawa 2521123 (JP)**

(74) Representative: **Novagraaf Technologies**
**Bâtiment O2**
**2, rue Sarah Bernhardt**
**CS90017**
**92665 Asnières-sur-Seine Cedex (FR)**

(56) References cited:
- **PERACAULA R ET AL: "Glycosylation of human pancreatic ribonuclease: Differences between normal and tumor states", GLYCOBIOLOGY, OXFORD UNIVERSITY PRESS, vol. 13, no. 4, 1 April 2003 (2003-04-01), pages 227-244, XP002362434, Oxford ISSN: 0959-6658, DOI: 10.1093/GLYCOB/CWG019**
- **R. PERACAULA ET AL.: "Human Pancreatic Ribonuclease 1", CANCER (AMERICAN CANCER SOCIETY), vol. 89, no. 6, 15 September 2000 (2000-09-15), pages 1252-1258, XP055232428, Atlanta GA USA**
- **DAISUKE N.: "Increased N-glycosylation of Asn88 in serum pancreatic ribonuclease 1 is a novel diagnostic marker for pancreatic cancer", SCIENTIFIC REPORTS (NATURE), vol. 4, no. 6715, 22 October 2014 (2014-10-22), pages 1-8, XP055232102, Basingstoke UK DOI: 10.1038/srep06715**
- **BARRABES S. ET AL.: 'Glycosylation of serum ribonuclease 1 indicates a major endothelial origin and reveals an increase in core fucosylation in pancreatic cancer.' GLYCOBIOLOGY vol. 17, no. 4, 2007, pages 388 - 400, XP002638711**

**(Cont. next page)**

- **FUTAMI J. ET AL.: 'Recombinant Human Pancreatic Ribonuclease Produced in E. coli : Importance of the Amino-Terminal Sequence.' BIOCHEM. BIOPHYS. RES. COMMUN. vol. 216, no. 1, 1995, pages 406 - 413, XP055104672**
- **Z. LIU ET AL: "GLP1-derived nonapeptide GLP1(28-36)amide protects pancreatic -cells from glucolipotoxicity", JOURNAL OF ENDOCRINOLOGY (BRITISH SOCIETY FOR ENDOCRINOLOGY), vol. 213, no. 2, 13 March 2012 (2012-03-13), pages 143-154, XP055353455, Bristol ISSN: 0022-0795, DOI: 10.1530/JOE-11-0328**

SEQUENCE LISTING

<110> TOSOH corporation

<120> Method for Detecting Cancer and Antibody Capable of Recognizing Pancreatic-Specific Ribonuclease 1

<130> AB634

<150> JP2012-131970
<151> 2012-06-11

<150> JP2012-272285
<151> 2012-12-13

<160> 6

<210> 1
<211> 468
<212> DNA
<213> Homo sapiens

<400> 1

```
atg gct ctg gag aag tct ctt gtc cgg ctc ctt ctg ctt gtc ctg ata    48
Met Ala Leu Glu Lys Ser Leu Val Arg Leu Leu Leu Leu Val Leu Ile
1               5                   10                  15
ctg ctg gtg ctg ggc tgg gtc cag cct tcc ctg ggc aag gaa tcc cgg    96
Leu Leu Val Leu Gly Trp Val Gln Pro Ser Leu Gly Lys Glu Ser Arg
            20                  25                  30
gcc aag aaa ttc cag cgg cag cat atg gac tca gac agt tcc ccc agc   144
Ala Lys Lys Phe Gln Arg Gln His Met Asp Ser Asp Ser Ser Pro Ser
        35                  40                  45
agc agc tcc acc tac tgt aac caa atg atg agg cgc cgg aat atg aca   192
Ser Ser Ser Thr Tyr Cys Asn Gln Met Met Arg Arg Arg Asn Met Thr
        50                  55                  60
cag ggg cgg tgc aaa cca gtg aac acc ttt gtg cac gag ccc ctg gta   240
Gln Gly Arg Cys Lys Pro Val Asn Thr Phe Val His Glu Pro Leu Val
65                  70                  75                  80
gat gtc cag aat gtc tgt ttc cag gaa aag gtc acc tgc aag aac ggg   288
Asp Val Gln Asn Val Cys Phe Gln Glu Lys Val Thr Cys Lys Asn Gly
                85                  90                  95
cag ggc aac tgc tac aag agc aac tcc agc atg cac atc aca gac tgc   336
Gln Gly Asn Cys Tyr Lys Ser Asn Ser Ser Met His Ile Thr Asp Cys
            100                 105                 110
cgc ctg aca aac ggc tcc agg tac ccc aac tgt gca tac cgg acc agc   384
Arg Leu Thr Asn Gly Ser Arg Tyr Pro Asn Cys Ala Tyr Arg Thr Ser
            115                 120                 125
ccg aag gag aga cac atc att gtg gcc tgt gaa ggg agc cca tat gtg   432
Pro Lys Glu Arg His Ile Ile Val Ala Cys Glu Gly Ser Pro Tyr Val
        130                 135                 140
cca gtc cac ttt gat gct tct gtg gag gac tct acc                   468
Pro Val His Phe Asp Ala Ser Val Glu Asp Ser Thr
145                 150                 155
```

<210> 2
<211> 384
<212> DNA
<213> Homo sapiens

<400> 2

```
aag gaa tcc cgg gcc aag aaa ttc cag cgg cag cat atg gac tca gac    48
Lys Glu Ser Arg Ala Lys Lys Phe Gln Arg Gln His Met Asp Ser Asp
1               5                   10                  15
agt tcc ccc agc agc agc tcc acc tac tgt aac caa atg atg agg cgc    96
Ser Ser Pro Ser Ser Ser Ser Thr Tyr Cys Asn Gln Met Met Arg Arg
                20                  25                  30
cgg aat atg aca cag ggg cgg tgc aaa cca gtg aac acc ttt gtg cac   144
Arg Asn Met Thr Gln Gly Arg Cys Lys Pro Val Asn Thr Phe Val His
            35                  40                  45
gag ccc ctg gta gat gtc cag aat gtc tgt ttc cag gaa aag gtc acc   192
Glu Pro Leu Val Asp Val Gln Asn Val Cys Phe Gln Glu Lys Val Thr
        50                  55                  60
tgc aag aac ggg cag ggc aac tgc tac aag agc aac tcc agc atg cac   240
Cys Lys Asn Gly Gln Gly Asn Cys Tyr Lys Ser Asn Ser Ser Met His
65                  70                  75                  80
atc aca gac tgc cgc ctg aca aac ggc tcc agg tac ccc aac tgt gca   288
Ile Thr Asp Cys Arg Leu Thr Asn Gly Ser Arg Tyr Pro Asn Cys Ala
                85                  90                  95
tac cgg acc agc ccg aag gag aga cac atc att gtg gcc tgt gaa ggg   336
Tyr Arg Thr Ser Pro Lys Glu Arg His Ile Ile Val Ala Cys Glu Gly
            100                 105                 110
agc cca tat gtg cca gtc cac ttt gat gct tct gtg gag gac tct acc   384
Ser Pro Tyr Val Pro Val His Phe Asp Ala Ser Val Glu Asp Ser Thr
            115                 120                 125
```

<210> 3
<211> 8
<212> PRT
<213> Homo sapiens

<400> 3

```
Arg Leu Thr Asn Gly Ser Arg Tyr
1               5
```

<210> 4
<211> 11
<212> PRT
<213> Homo sapiens

<400> 4

```
Arg Leu Thr Asn Gly Ser Arg Tyr Pro Asn Cys
1               5                   10
```

<210> 5
<211> 12
<212> PRT
<213> Homo sapiens

<400> 5

```
          Ser Arg Leu Thr Asn Gly Ser Arg Tyr Pro Asn Cys
          1               5               10
```

<210> 6
<211> 12
<212> PRT
<213> Homo sapiens

<400> 6

```
          Ser Arg Leu Thr Asp Gly Ser Arg Tyr Pro Asn Cys
          1               5               10
```

**Description**

BACKGROUND ART

The present invention relates to a method for detecting pancreatic cancer and an antibody that recognizes pancreatic ribonuclease 1. More particularly, the present invention relates to a method for detecting pancreatic cancer by measuring the presence or absence of a glycan chain linked to a site capable of being modified by an N-glycan chain in a glycoprotein.

BACKGROUND ART

Methods used to detect cancer at the stage of early diagnosis preferably use a non-invasive biological sample in the manner of a comparatively easily collectible body fluid such as blood or urine as a specimen. Examples of serum markers currently used when diagnosing pancreatic cancer include CA19-9 and DUPAN-2. However, these markers have the shortcoming of preventing a definitive diagnosis due to such factors as low pancreas specificity and the inability of the markers to react for genetic reasons.

Pancreatic ribonuclease 1, which is a member of the ribonuclease family (and is referred to as "RNase 1"), is a glycoprotein that is specifically expressed in the pancreas and is secreted into extracellular body fluid. This protein is translated as a peptide composed of 156 amino acids (SEQ ID NO: 1), and is secreted outside the cells as a mature glycoprotein having a peptide sequence consisting of 128 amino acids (SEQ ID NO: 2) after having been removed of the secretion signal and glycosylated. Sites capable of being modified by an N-glycan chain, namely those amino acid residues capable of being modified by an N-glycan chain, consist of asparagine residues at positions 34, 76 and 88 in SEQ ID NO: 2.

Pancreatic RNase 1 has long been a subject of research, and although changes in its expression and activity during cancer have been reported, there have no actual cases of its clinical application. Although Doran et al. reported in 1980 that ribonuclease activity in serum increases in pancreatic cancer patients (Non-Patent Document 1), the report only provides a description of activity, and findings relating to the molecular species of that ribonuclease are not specified. With respect to glycan chain modification of pancreatic RNase 1, a report was released in 1994 relating to ribonuclease obtained from healthy individuals, and although there are reports regarding the degree of glycan chain addition at each of the three putative glycosylation sites, there have been no reports describing their correlation with pancreatic cancer (Non-Patent Document 2). Fernandez-Salas, et al. reported in 2000 that the molecular weight of pancreatic RNase 1 secreted from pancreatic cancer-derived cultured cells was larger than that of RNase 1 obtained from the normal pancreas, and indicated that one of the reasons for this was an increase in the amount of glycan chain modification (Non-Patent Document 3). Moreover, the same research group published reports in 2003 and 2007 relating to glycan chain modification of pancreatic RNase 1, and in those reports, indicated that there is a change in the structure of N-glycan chains of pancreatic RNase 1 obtained from the serum of pancreatic cancer patients, clearly demonstrated an increase in core fucosylated, biantennary complex glycan chains in particular, and determined that the increase in molecular weight of pancreatic RNase 1 is attributable to an increase in the molecular weight per se of the added glycan chain structure (Non-Patent Documents 4 and 5). Futami et al. reports an antibody specific to human RNase 1 but is silent in respect of "N-glycosylation site in the pancreatic ribonuclease 1 linked with an N-glycan chain", (Non-Patent Document 6).Peracaula et al. reports that RNase 1 is expressed in most pancreatic adenocarcinomas (Non-Patent Document 7). However, there have yet to be any reports demonstrating a correlation between cancer and the presence or absence of glycan chain linked to putative N-glycosylation sites of glycoproteins.Prior Art Documents

Non-Patent Documents

Non-Patent Document 1: J. Clin. Pathol. 33, 1212-13 (1980)
Non-Patent Document 2: Biol. Chem. Hoppe Seyler 375, 357-63 (1994)
Non-Patent Document 3: Eur. J. Biochem. 267, 1484-1494 (2000)
Non-Patent Document 4: Glycobiology 13, 227-244 (2003)
Non-Patent Document 5: Glycobiology 17, 388-400 (2007)
Non-Patent Document 6 : Biochem. Biophys. Res., 26(1): 406-413 (1995)
Non-Patent Document 7: Cancer, 89(6): 1252-1258 (2003)

## DISCLOSURE OF THE INVENTION

Problems to be Solved by the Invention

An object of the present invention is to provide a method for detecting pancreatic cancer and an antibody that recognizes pancreatic RNase 1 by focusing on putative N-glycosylation sites of a glycoprotein.

Means for Solving the Problems

As a result of conducting extensive studies to solve the aforementioned problems of the present invention, the inventor of the present invention found that cases in which glycan chains are linked to putative N-glycosylation sites of pancreatic RNase 1 increase in pancreatic cancer patients in comparison with healthy individuals, thereby leading to completion of the present invention. In addition, the inventor of the present invention also found an antibody that specifically binds to pancreatic RNase 1 and uses a putative N-glycosylation site of pancreatic RNase 1 as a portion of the antigen recognition site thereof, and in which binding to pancreatic RNase 1 is inhibited in the case a glycan chain binds to the putative N-glycosylation site, thereby leading to completion of the present invention.
Namely, the present invention is as indicated below.

(1) A method for detecting pancreatic cancer in a human subject characterized by measuring the amount of either a putative N-glycosylation site of the pancreatic RNase 1 linked with an N-glycan chain or not linked with an N-glycan chain,
wherein the putative N-glycosylation site is one or more asparagine residues selected from asparagine at positions 34, 76 and 88 of SEQ ID NO: 2, and wherein the amount of the site linked with the N-glycan chain is increased in pancreatic cancer patients in comparison with healthy individuals and the amount of the site not linked with the N-glycan chain is decreased in pancreatic cancer patients in comparison with healthy individuals.
(2) A method for detecting pancreatic cancer in a human subject that includes determining the ratio A/B for A and B indicated below:

A: amount of putative N-glycosylation site of either the pancreatic RNase 1 linked with an N-glycan chain or not linked with N-glycan chain; and,
B: amount of putative N-glycosylation site of the pancreatic RNase 1,

wherein the putative N-glycosylation site is one or more asparagine residues selected from asparagine at positions 34, 76 and 88 of SEQ ID NO: 2; and wherein when A represents the amount of the site not linked with an N-glycan chain, the value of A/B is lower in pancreatic cancer patients in comparison with healthy individuals; and
when A represents the amount of the site linked with an N-glycan chain, the value of A/B is higher in pancreatic cancer patients in comparison with healthy individuals.
(3) The method according to any of (1) to (2),
wherein the putative N-glycosylation site is an asparagine residue at position 88 of SEQ ID NO: 2.
(4) The method according to any of (1) to (2),
wherein the putative N-glycosylation site is an asparagine residue at position 76 of SEQ ID NO: 2.
(5) The method according to any of (1) to (2),
wherein the putative N-glycosylation site is an asparagine residue at position 34 of SEQ ID NO: 2.
(6) A monoclonal antibody, or fragment thereof, both of which recognize a putative N-glycosylation site of the antigen recognition site of human pancreatic RNase 1, wherein the putative N-glycosylation site is one asparagine residue selected from asparagine at positions 34, 76 and 88 of SEQ ID NO: 2, and wherein binding to the putative N-glycosylation site of human pancreatic RNase 1 occurs in the case that the site is not linked with an N-glycan chain, and is statistically significantly inhibited in the case that the site is linked with an N-glycan chain.
(7) The monoclonal antibody, or fragment thereof, according to (6), wherein the antigen recognition site contains the putative N-glycosylation site being the asparagine residue at position 88 of SEQ ID NO: 2.
(8) A monoclonal antibody or fragment thereof, both of which are capable of binding to human pancreatic RNase 1 simultaneously with the binding to human pancreatic RNase 1 of the monoclonal antibody, or fragment thereof, according to any of (6) to (7) above.
(9) The method according to any of (1) to (5) that comprises contacting the monoclonal antibody or fragment thereof according to (6) or (7) described above with a sample, and measuring the amount of pancreatic RNase 1 that has formed a complex with said monoclonal antibody or fragment thereof.
(10) The method according to (9) that comprises further contacting the monoclonal antibody or fragment thereof according to (8) with the sample previously contacted by the monoclonal antibody or fragment thereof according to (6) or (7), and measuring the amount of pancreatic RNase 1 that has formed a complex with said two monoclonal antibodies or fragments thereof.

(11) The method according to (9) or (10) above that comprises a first step to contact the sample with the monoclonal antibody or fragment thereof according to (6) or (7) or with the monoclonal antibody or fragment thereof according to (8), and a second step to further contact the sample obtained in the first step, respectively, with the monoclonal antibody or fragment thereof according to (8) or with the monoclonal antibody or fragment thereof according to (6) or (7).

(12) A pharmaceutical comprising the monoclonal antibody, or fragment thereof, according to any of (6) to (7) above.

(13) The method according to any of (1) to (5), wherein the amount of the putative N-glycosylation site of the pancreatic RNase 1 linked with an N-glycan chain or not linked with an N-glycan chain is determined by mass spectrometry.

(14) The method according to (13) above, wherein determining by mass spectrometry includes decomposing the pancreatic ribonuclease 1 with an enzyme and then measuring the mass of the resulting peptide fragment and/or glycopeptide fragment.

The following provides a more detailed explanation of the present invention. The present invention detects pancreatic cancer in a human subject by measuring the amount of a putative N-glycosylation site in the pancreatic ribonuclease 1 linked with an N-glycan chain or not linked with an N-glycan chain. At this time, the amount of the putative N-glycosylation "site" in the pancreatic ribonuclease 1 linked with the N-glycan chain or the amount of the "site" not linked with an N-glycan chain is measured, while the amount of a "glycan chain" linked to the putative N-glycosylation site is not measured.

The pancreatic RNase 1 is derived from a human biological sample. As a result of using this as a measurement target, human pancreatic cancer can be detected. At this time, since there are many cases in which an N-glycan chain is linked to a putative N-glycosylation site in pancreatic cancer patients in comparison with healthy individuals, and there is an increase in the amount of the putative N-glycosylation site linked with the N-glycan chain, pancreatic cancer can be detected by using this as an indicator.

In addition, the present invention is a method for detecting pancreatic cancer in a human subject that includes the determination of A/B for A and B as indicated below:

A: amount of putative N-glycosylation site of either the pancreatic ribonuclease 1 linked with an N-glycan chain or not linked with an N-glycan chain; and,

B: amount of putative N-glycosylation site of the pancreatic ribonuclease 1,

wherein the putative N-glycosylation site is one or more asparagine residues selected from asparagine at positions 34, 76 and 88 of SEQ ID NO: 2; and wherein

when A represents the amount of the site not linked with an N-glycan chain, the value of A/B is lower in pancreatic cancer patients in comparison with healthy individuals; and

when A represents the amount of the site linked with an N-glycan chain, the value of A/B is higher in pancreatic cancer patients in comparison with healthy individuals.

In addition, a method is preferably used in which A preferably represents the amount of the site not linked with an N-glycan chain, and the value of A/B is low in pancreatic cancer patients in comparison with healthy individuals. In addition, a method is also preferably used in which A represents the amount of the site where N-glycan chain is bound, and the value of A/B is high in pancreatic cancer patients in comparison with healthy individuals.

In the case B represents the amount of a putative N-glycosylation site of pancreatic RNase 1, there are no particular limitations on the determination method thereof, and for example, the amount of pancreatic RNase 1 can be determined followed by converting that value and using as the value of B. More specifically, since pancreatic RNase 1 has three putative N-glycosylation sites (asparagine residues at positions 34, 76 and 88 of SEQ ID NO: 2) as previously described, when the amount of any one, two or all of these sites is used as the measurement target, the amount of the putative N-glycosylation site of pancreatic RNase 1 represented by B can be respectively converted to 1 time, 2 times or 3 times the amount of pancreatic RNase 1 corresponding thereto. Furthermore, the amount of pancreatic RNase 1 can be determined by method such as that using an immunological assay method or mass spectrometry.

Furthermore, pancreatic RNase 1 has three putative N-glycosylation sites (asparagine residues at positions 34, 76 and 88 of SEQ ID NO: 2) as previously described. Pancreatic cancer in human subject can be detected by determining either the amount of the site linked with the N-glycan chain or the amount of the site not linked with an N-glycan chain among one, two or all of those sites, or by determining the ratio between that amount and the amount of the putative N-glycosylation site of pancreatic RNase 1. Since a remarkable difference in values is observed between pancreatic cancer patients and healthy individuals if the aforementioned amount is measured as the amount of the putative N-glycosylation site with respect to the asparagine residue at position 88 of SEQ ID NO: 2 in particular, this is preferable as a method for detecting pancreatic cancer. Similarly, it is also preferable to measure the aforementioned amount for the asparagine residue at position 76 or position 34 of SEQ ID NO: 2.

In addition, the present invention is a monoclonal antibody, or fragment thereof, both of which recognize a putative N-glycosylation site of the antigen recognition site of human pancreatic RNase 1. Such a monoclonal antibody can be produced in accordance with normal methods by using human pancreatic RNase 1, or a vicinity peptide sequence that contains a putative N-glycosylation site thereof, as an immunogen. In addition, antigen specificity of the antibody can be determined by analyzing binding of the antibody to an antigen-determining group thereof using a standard assay such as ELISA or FACS. In addition, examples of fragments of the monoclonal antibody include Fab or F(ab')$_2$ fragments obtained by digesting the entire antibody with various enzymes. In the present invention, among such monoclonal antibodies or fragments thereof, that in which binding to a putative N-glycosylation site of human pancreatic RNase 1 occurs in the case that a glycan chain is not linked, but is inhibited in the case that an N-glycan chain is linked, is preferable.

Moreover, among such monoclonal antibodies or fragments thereof in the present invention, that in which the antigen recognition site is a region containing the asparagine residue at position 88 of SEQ ID NO: 2 is preferable. A monoclonal antibody, or fragment thereof, which contains the amino acid sequence among the putative glycosylation sites of pancreatic RNase 1 located closest to the carboxyl terminal in a portion of the antigen recognition site thereof, is particularly preferable. An example of such a monoclonal antibody is anti-human pancreatic RNase 1 monoclonal antibody RrhRN0723 produced in the present invention. Binding of this monoclonal antibody RrhRN0723 to human pancreatic RNase 1 is inhibited in the case that an N-glycan chain is linked to the asparagine residue at position 88 of SEQ ID NO: 2 among the putative N-glycosylation sites of human pancreatic RNase 1.

In addition, the present invention is a monoclonal antibody or fragment thereof both of which are capable of binding to human pancreatic RNase 1 simultaneously with the binding of a monoclonal antibody or fragment thereof both of which recognizing a putative N-glycosylation region of the antigen recognition site of human pancreatic RNase 1. A monoclonal antibody that uses, for example, human pancreatic RNase 1 as an immunogen, can be produced in accordance with ordinary methods, and is capable of binding to human pancreatic RNase 1 simultaneously with the binding of the aforementioned monoclonal antibody, or fragment thereof, both of which recognize a putative N-glycosylation site of the antigen recognition site of human pancreatic RNase 1, may be screened for use as such monoclonal antibody. In addition, examples of fragments of the monoclonal antibody include Fab or F(ab')$_2$ fragments obtained by digesting the entire antibody with various enzymes. An example of such a monoclonal antibody is anti-human pancreatic RNase 1 monoclonal antibody MrhRN0614 produced in the present invention. This monoclonal antibody MrhRN0614 is capable of binding to human pancreatic RNase 1 simultaneously with the binding of the aforementioned monoclonal antibody RrhRN0723.

In the present invention, such a monoclonal antibody or fragment thereof may be labeled with alkaline phosphatase, peroxidase, biotin or fluorescein isothiocyanate and the like.

In addition, the present invention is a method for detecting pancreatic cancer in a human subject that comprises contacting a monoclonal antibody or fragment thereof, both of which recognize a putative N-glycosylation site of the antigen recognition site of human pancreatic RNase 1, with a sample, and measuring the amount of pancreatic RNase 1 that has formed a complex with the monoclonal antibody or fragment thereof. An example of such a method is a competitive method or antibody array method.

In addition, the present invention is a method for detecting pancreatic cancer in a human subject that comprises further contacting a monoclonal antibody or fragment thereof that is capable of binding to pancreatic RNase 1 simultaneously with the binding of the monoclonal antibody or fragment thereof, both of which recognize a putative N-glycosylation site of the antigen recognition site of human pancreatic RNase 1 , with a sample previously contacted by the aforementioned monoclonal antibody or fragment thereof, both of which recognize a putative N-glycosylation site of the antigen recognition site of human pancreatic RNase 1, and measuring the amount of the putative N-glycosylation site of pancreatic RNase 1 that has formed a complex with said two monoclonal antibodies or fragments thereof, linked with an N-glycan chain or not linked with an N-glycan chain. At this time, although said two antibodies or fragments thereof, may be simultaneously contacted with the sample, they are preferably contacted sequentially. In the case of contacting sequentially, the method comprises a first step to contact one of the two monoclonal antibodies with the sample, followed by a second step to contact the other one of the two monoclonal antibodies with the sample obtained in the first step. At this time, the sample is preferably contacted with an antibody that is capable of binding to pancreatic RNase 1 simultaneously with the binding of the monoclonal antibody or fragment thereof, both of which recognize a putative N-glycosylation site of the antigen recognition site of human pancreatic RNase 1 in the first step and then contacted with an antibody that recognizes a putative N-glycosylation site of the antigen recognition site of human pancreatic RNase 1 in the second step. The aforementioned monoclonal antibody, or fragment thereof, of the present invention can be used, and more preferably, monoclonal antibody RrhRN0723, or a fragment thereof, is used for an antibody that recognizes a putative N-glycosylation site of the antigen recognition site of human pancreatic RNase 1 and monoclonal antibody MrhRN0614, or a fragment thereof, is used for an antibody capable of binding to human pancreatic RNase 1 simultaneously with the binding of the monoclonal antibody or fragment thereof, both of which recognize a putative N-glycosylation site of the antigen recognition site of human pancreatic RNase 1. Examples of such a method include ELISA and EIA as well as methods comprising isolating and measuring a formed immune complex by liquid chromatography, high-performance liquid chromatography or immunochromatography and the like.

A monoclonal antibody, or fragment thereof, both of which recognize a putative N-glycosylation site of the antigen recognition site of human pancreatic RNase 1 in this manner can be used as a diagnostic drug or other type of pharmaceutical.

In the present invention, there are no particular limitations on the method used to determine the amount of putative N-glycosylation site of the pancreatic ribonuclease 1 linked with an N-glycan chain or not linked with an N-glycan chain, and the amount may be determined by a method that uses an immunoassay as previously described or the amount may be determined by mass spectrometry. In the case of determining by mass spectrometry, the amount of putative N-glycosylation site of the pancreatic ribonuclease 1 linked with an N-glycan chain or not linked with an N-glycan chain can be determined by, for example, decomposing the pancreatic ribonuclease 1 with an enzyme and the like and then measuring the mass of the resulting peptide fragment and/or glycopeptide fragment with a mass spectrometer and the like. In the case of a method that uses a mass spectrometer, examples of methods for obtaining a peptide fragment from the pancreatic ribonuclease 1 include a series of deglycosylation treatments using glycosidase and fragmentation of the peptide backbone of a protein with endopeptidase as reported in J. Proteome Res. 3, 556-566 (2004). An example of a method of deglycosylation treatment by glycosidase comprises using one or more exoglycosidases selected from the group of consisting of sialidase, galactosidase, hexosaminidase, mannosidase and fucosidase, and an endoglycosidase such as endoglycosidase H that acts on the chitobiose structure of the basic structure of N-glycan chains, and allowing the monosaccharide structure of N-acetylglucosamine to retain on the asparagine residue of the putative N-glycosylation site. In this method, since the monosaccharide structure of N-acetylglucosamine remains on the peptide backbone of the protein, a glycopeptide fragment, for which mass has increased from the estimated molecular weight determined by calculation from the amino acid sequence by the amount of the molecular weight of N-acetylglucosamine, can be detected among fragments obtained by arbitrary endopeptidase treatment, thereby making it possible to identify a state in which a glycan chain has been added to a specific putative N-glycosylation site. The presence or absence of the addition of a glycan chain can then be confirmed by mass spectrometry by analyzing pancreatic RNase 1 using any of the examples of methods previously described.

Effects of the Invention

According to the present invention, pancreatic cancer can be detected. In addition, the present invention provides a monoclonal antibody that recognizes a putative N-glycosylation site of the antigen recognition site of human pancreatic RNase 1, and a monoclonal antibody capable of binding to pancreatic RNase 1 simultaneously with the binding of that monoclonal antibody. The aforementioned pancreatic cancer can be detected by using these two antibodies.

BRIEF DESCRIPTION OF THE DRAWINGS

FIG. 1 is a graph indicating the results of measuring binding ability of anti-human pancreatic RNase 1 antibodies to antigens introduced with a glycan-modification deficient mutation in Example 2.

FIG. 2 is a graph indicating the results of measuring the binding ability of anti-human pancreatic RNase 1 antibodies to antigens introduced with amino acid substitution mutations in Example 2.

FIG. 3 is a diagram indicating the results of analyzing the states of glycan modification of antigen m001 introduced with a glycan-modification deficient mutation in Example 2.

FIG. 4 is a graph showing the results of measuring the concentrations of alpha-methylmannoside in various fractions and the binding ability of anti-human pancreatic RNase 1 antibodies in Example 2.

FIG. 5 is a diagram indicating the results of measuring glycan chain-modified pancreatic RNase 1 in various specimens in Example 3.

FIG. 6 is a graph indicating a significant difference between the distribution of measured values for pancreatic cancer and non-pancreatic cancer in Example 3.

FIG. 7 is a graph indicating the results of an ROC statistical analysis of pancreatic cancer and non-pancreatic cancer in Example 3.

FIG. 8 is a diagram indicating the results of examining the antigen recognition performance of anti-human pancreatic RNase 1 peptide antibodies as determined by western blotting in Example 4.

FIG. 9 is a diagram indicating the results of investigating the presence or absence of glycan chain linked to Asn88 in pancreatic RNase 1 in the sera of healthy individuals and pancreatic cancer patients as determined by western blotting using anti-human pancreatic RNase 1 peptide antibodies in Example 4.

FIG. 10 is a graph indicating the results of measuring binding ability of anti-human pancreatic RNase 1 antibody RrhRN1111 to antigens introduced with a glycan-modification deficient mutation in Example 5.

FIG. 11 is a diagram indicating the results of determining $F_3/t$ values and $G_3/t$ values from measured values of RNase 1 in healthy individuals and pancreatic cancer patients in Example 5.

FIG. 12 is a graph indicating the results of measuring a dilution series of a competing antigen by an antigen immobilized competition method using RrhRN0723 antibody in Example 6.

Examples

Example 1 - Antibody Production

Immunogen Preparation

In order to acquire a polypeptide containing the full length of human pancreatic RNase 1, an expression plasmid was produced in which a gene sequence encoding mature human pancreatic RNase 1 (SEQ ID NO: 2) was inserted into a plasmid vector capable of expressing in insect cells. More specifically, a gene sequence encoding a human immunoglobulin kappa chain, a gene sequence encoding His tag, a gene sequence encoding FLAG tag, and a region (SEQ ID NO: 2) in which a gene of a signal peptide in the form of 84 nucleic acid residues encoding amino acid no. 1 to no. 28 was removed from a gene sequence encoding human pancreatic RNase 1 (SEQ ID NO: 1) were inserted into a multicloning site of an insect cell recombinant protein expression plasmid, pIZ/V5His Vector (Life Technologies Corp.) in order from 5' upstream side. As a result of inserting the resulting expression plasmid pIZ-KFH-hRNase1 into insect cell line Sf9 using Cellfectin II (Life Technologies Corp.), recombinant human pancreatic RNase 1, in which human immunoglobulin kappa chain was added to the N-terminal side thereof, was confirmed to be secreted into the medium. The protein secreted into the medium was concentrated and purified from the culture supernatant by affinity purification using anti-human immunoglobulin kappa light chain antibody to obtain an immunogen.

Immunization of Immune Animals

Mice and rats were immunized using the aforementioned immunogen. More specifically, in the case of immunizing mice, 100 $\mu$g of immunogen were administered into the abdominal cavities of 6-week-old Balb/c mice together with Freund's complete adjuvant for the purpose of initial immunization. Subsequently, 100 $\mu$g of immunogen were administered into the abdominal cavities of the mice 7, 14, 21, 28 and 35 days later together with Freund's incomplete adjuvant for the purpose of booster immunization. Moreover, 100 $\mu$g of immunogen were administered into the abdominal cavities of the mice 42 days later together with physiological saline for the purpose of final immunization. In the case of immunizing rats, 100 $\mu$g of immunogen were administered into the foot pads of both back legs of 6-week-old WHY female rats together with Freund's complete adjuvant for the purpose of initial immunization. Subsequently, 100 $\mu$g of immunogen were administered into the foot pads of the back legs 28 days later together with physiological saline for the purpose of final immunization.

Production of Antibody-Producing Hybridomas

The spleens of the mice were excised 3 days after final immunization and spleen cells were recovered. Iliac lymph nodes and inguinal lymph nodes were excised from the rats to obtain lymph node cells. After respectively fusing the mouse spleen cells and rat lymph node cells to a mouse myeloma cell line by electrofusion, hybridomas were selected by inoculating onto a 96-well cell culture plate using GIT medium (Wako Pure Chemical Industries Co., Ltd.) containing hypoxanthine, aminopterin and thymidine.

Selection of hybridoma Line Producing Mouse Anti-Human Pancreatic RNase 1 Antibody

A hybridoma line producing mouse anti-human pancreatic RNase 1 antibody was selected by screening according to the ELISA method using as an indicator reactivity of antibody secreted by the hybridomas into the medium to recombinant human pancreatic RNase 1. The ELISA procedure used for screening was as indicated below. 50 $\mu$l of phosphate buffer (50 mM sodium phosphate, 150 mM NaCl, pH 7.4) containing 25 ng of goat anti-human immunoglobulin kappa chain antibody (Sigma-Aldrich Corp.) were added to each well of a 96-well microtiter plate (Greiner Bio-One Co., Ltd.) followed by fixing for 16 hours at 4°C. After washing the wells three times with 300 $\mu$l of washing solution (20 mM Tris-HCl, 150 mM NaCl, pH 7.4), 200 $\mu$l of blocking solution containing 3% BSA (3% BSA, 20 mM Tris-HCl, 150 mM NaCl, pH 7.4) were added followed by allowing to stand for 2 hours at room temperature to carry out blocking (anti-human immunoglobulin kappa chain antibody-immobilized plate). After washing each well three times with 300 $\mu$l of

washing solution, recombinant human pancreatic RNase 1 diluted to 0.5 μg/ml with a diluent (1% BSA, 20 mM Tris-HCl, 150 mM NaCl, 0.05% Tween-20, pH 7.4) was added followed by allowing to stand for 1 hour at room temperature. After washing each well three times with 300 μl of washing solution containing a surfactant (20 mM Tris-HCl, 150 mM NaCl, 0.05% Tween-20, pH 7.4), 50 μl of hybridoma culture supernatant were added followed by allowing to stand for 1 hour at room temperature. Next, after washing each well three times with 300 μl of washing solution containing surfactant, 50 μl of a diluted solution containing 0.01 μg of anti-mouse IgG antibody (Rockland Inc.) labeled with horseradish peroxidase (HRP) were added followed by allowing to stand for 1 hour at room temperature. Finally, after washing each well three times with 300 μl of washing solution containing surfactant, 50 μl of tetramethylbenzidine (TMB) solution (KPL Inc.) were added followed by coloring for 15 minutes, terminating the reaction by adding 1 M phosphoric acid solution and measuring absorbance at 450 nm. Based on the results of screening, hybridomas were obtained that produced antibodies exhibiting strong affinity for pancreatic RNase 1. The resulting hybridomas were subjected to limiting dilution to obtain monoclonal antibody MrhRN0614.

Selection of Hybridoma Line Producing Rat Anti-Human Pancreatic RNase 1 Antibody

A hybridoma line producing rat anti-human pancreatic RNase 1 antibody was selected by screening according to the ELISA method using as an indicator reactivity of antibody secreted by the hybridomas into the medium to pancreatic RNase 1 derived from human pancreatic cancer cells (Capan 1). The ELISA procedure used for screening was as indicated below. 50 μl of phosphate buffer (50 mM sodium phosphate, 150 mM NaCl, pH 7.4) containing 25 ng of mouse anti-human pancreatic RNase 1 antibody (MrhRN0614) were added to each well of a 96-well microtiter plate (Greiner Bio-One Co., Ltd.) followed by fixing for 16 hours at 4°C. After washing the wells three times with 300 μl of washing solution (20 mM Tris-HCl, 150 mM NaCl, pH 7.4), 200 μl of blocking solution containing 3% BSA (3% BSA, 20 mM Tris-HCl, 150 mM NaCl, pH 7.4) were added followed by allowing to stand for 2 hours at room temperature to carry out blocking (anti-human pancreatic RNase 1 antibody-immobilized plate). After washing each well three times with 300 μl of washing solution (20 mM Tris-HCl, 150 mM NaCl, pH 7.4), a culture supernatant of human pancreatic cancer cells (Capan 1) diluted two-fold with a diluent (1% BSA, 20 mM Tris-HCl, 150 mM NaCl, 0.05% Tween-20, pH 7.4) was added followed by allowing to stand for 1 hour at room temperature. After washing each well three times with 300 μl of washing solution containing a surfactant (20 mM Tris-HCl, 150 mM NaCl, 0.05% Tween-20, pH 7.4), 50 μl of hybridoma culture supernatant were added followed by allowing to stand for 1 hour at room temperature. Next, after washing each well three times with 300 μl of washing solution containing surfactant, 50 μl of a diluted solution containing 0.01 μg of HRP-labeled anti-rat IgG antibody (American Qualex Scientific Products) were added followed by allowing to stand for 1 hour at room temperature. Finally, after washing each well three times with 300 μl of washing solution containing surfactant, 50 μl of TMB solution were added followed by coloring for 15 minutes, terminating the reaction by adding 1 M phosphoric acid solution and measuring absorbance at 450 nm. Based on the results of screening, hybridomas were obtained that produced antibodies exhibiting strong affinity for pancreatic RNase 1. The resulting hybridomas were subjected to limiting dilution to obtain monoclonal antibody RrhRN0723.

Example 2 - Measurement of Antibody Specificity Preparation of Recombinant Human Pancreatic RNase 1 using Mammalian Expression System

In order to acquire a polypeptide containing the full length of human pancreatic RNase 1 in mammalian cells, an expression vector was produced by inserting a gene sequence encoding human pancreatic RNase 1 (SEQ ID NO: 2) into a pcDNA3.1-mycHis vector (Life Technologies Corp.). More specifically, a portion of a gene sequence encoding a recombinant protein of the insect cell expression plasmid (pIZ-KFH-hRNase1) produced in order to prepare an immunogen was inserted into a pcDNA3.1-mycHis vector (Life Technologies Corp.) by a molecular biological technique to produce pcDNA-KFH-hRNase1. As a result of introducing the resulting mammalian cell expression plasmid into a cultured cell line derived from Chinese hamster ovary (to be referred to as line CHO-K1) using Lipofectamine 2000 (Life Technologies Corp.), recombinant human pancreatic RNase 1 having a human immunoglobulin kappa chain added to the N-terminal side thereof was confirmed to be secreted into the medium. The protein secreted into the medium was concentrated and purified from the culture supernatant by affinity purification using anti-human immunoglobulin kappa light chain antibody to acquire recombinant human pancreatic RNase 1. The purified recombinant human pancreatic RNase 1 was used in an experiment to determine antibody specificity.

Preparation of Glycan-Modification Deficient Mutant Antigens

Plasmids expressing a glycan-modification deficient mutant antigen were produced by repeatedly introducing amino acid substituted mutation by PCR mutagenesis using the expression vector (pcDNA-KFH-hRNase1) produced in the previous section as template. PCR mutagenesis was carried out using the PrimeSTAR Mutagenesis Basal Kit (Takara Bio Inc.) in accordance with the manual provided. More specifically, a plasmid expressing glycan-modification deficient mutant recombinant human pancreatic RNase 1 was produced by substituting the amino acid residue at position 3 of the consensus sequence of the putative N-glycosylation site (Asn-Xaa-Ser/Thr, wherein Xaa represents an amino acid residue other than proline) with an amino acid other than a serine residue or threonine residue (Table 1). The resulting expression plasmid introduced with the glycan-modification deficient mutation was introduced into line CHO-K1 using Lipofectamine 2000 (Life Technologies Corp.) to confirm that glycan-modification deficient mutant recombinant human pancreatic RNase 1, having human immunoglobulin kappa chain added to the N-terminal side thereof, is secreted into the medium. The glycan-modification deficient mutant recombinant human pancreatic RNase 1 secreted into the medium was used in an experiment to determine antibody specificity.

[Table 1]

| Mutant | Amino Acid Mutation Introducing Site | Possibility of Glycan Chain Modification | | |
|---|---|---|---|---|
| | | Asn34 | Asn76 | Asn88 |
| WT | Mutation-free | Yes | Yes | Yes |
| m011 | Thr36→Ala | No | Yes | Yes |
| m101 | Ser78→Ala | Yes | No | Yes |
| m110 | Ser90→Ala | Yes | Yes | No |
| m001 | Thr36→Ala, Ser78→Ala | No | No | Yes |
| m010 | Thr36→Ala, Ser90→Ala | No | Yes | No |
| m100 | Ser78→Ala, Ser90→Ala | Yes | No | No |
| m000 | Thr36→Ala, Ser78→Ala, Ser90→Ala | No | No | No |
| m000-N88D | Thr36→Ala, Ser78→Ala, Asn88→Asp | No | No | No |

Measurement of Reactivity of Anti-Human Pancreatic RNase 1 Antibodies to Glycan-Modification Deficient Mutant Antigens

Reactivity of anti-human pancreatic RNase 1 antibodies to glycan-modification deficient mutant recombinant human pancreatic RNase 1 was measured by sandwich ELISA as described below. 50 $\mu$l of phosphate buffer (50 mM sodium phosphate, 150 mM NaCl, pH 7.4) containing 25 ng of goat anti-human immunoglobulin kappa chain antibody (Sigma-Aldrich Corp.) were added to each well of a 96-well microtiter plate (Greiner Bio-One Co., Ltd.) followed by fixing for 16 hours at 4°C. After washing the wells three times with 300 $\mu$l of washing solution (20 mM Tris-HCl, 150 mM NaCl, pH 7.4), 200 $\mu$l of TBS solution containing 3% BSA were added followed by allowing to stand for 2 hours at room temperature to carry out blocking (anti-human immunoglobulin kappa chain antibody-immobilized plate). After washing each well three times with 300 $\mu$l of washing solution, a culture supernatant of CHO-KI cells introduced with the aforementioned expression plasmid containing a glycan-modification deficient mutation was added followed by allowing to stand for 1 hour at room temperature. After washing each well three times with 300 $\mu$l of washing solution containing a surfactant (20 mM Tris-HCl, 150 mM NaCl, 0.05% Tween-20, pH 7.4), 50 $\mu$l of a diluted solution containing 25 ng of HRP-labeled mouse anti-human pancreatic RNase 1 antibody (MrhRN0614) or 25 ng of HRP-labeled rat anti-human pancreatic RNase 1 antibody (RrhRN0723) were added followed by allowing to stand for 1 hour at room temperature. Finally, after washing each well three times with 300 $\mu$l of washing solution containing surfactant, 50 $\mu$l of TMB solution were added followed by coloring for 10 minutes, terminating the reaction by adding 1 M phosphoric acid solution and measuring absorbance at 450 nm.

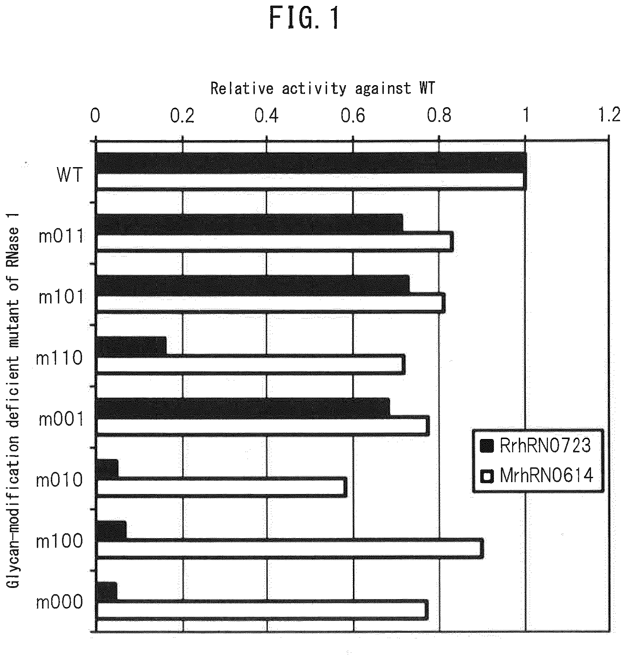

The results of the measurements are shown in FIG. 1. Relative values based on a value of 1.0 for the amount of each antibody bound to the mutation-free recombinant antigen WT are plotted on the horizontal axis. Each of the glycan-modification deficient mutant antigens are shown on the vertical axis. The horizontal solid black bars indicate the bound amounts of RrhRN0723 antibody, while the horizontal white bars indicate the bound amounts of MrhRN0614 antibody. Since the reactivity of RrhRN0723 antibody to mutants in which the glycan chain is no longer modified at asparagine at position 88 as a result of substituting the serine residue at positon 90 for alanine (m110, m010, m100 and m000) is lower than the reactivity of MrhRN0614 antibody, rat anti-human pancreatic RNase 1 antibody (RrhRN0723) was determined to recognize the vicinity of the putative glycosylation site at the asparagine residue at position 88. In addition, since mouse anti-human pancreatic RNase 1 antibody (MrhRN0614) maintains the same level of activity as the mutation-free recombinant antigen WT regardless of the glycan-modification deficient mutant antigen, it was determined to recognize pancreatic RNase 1 independent of the putative glycosylation site.

Preparation of Amino Acid Substitution Mutant Antigens

Plasmids expressing antigens introduced with an amino acid substitution deletion were produced by introducing amino acid substituted mutants by PCR mutagenesis using the aforementioned expression vector (pcDNA-KFH-hRNase1) as template. PCR mutagenesis was carried out using the PrimeSTAR Mutagenesis Basal Kit (Takara Bio Inc.) in accordance with the manual provided. More specifically, plasmids expressing amino acid substitution mutant recombinant human pancreatic RNase 1 were produced by respectively substituting amino acid residues from the amino acid at position 85 to the amino acid at positon 92 in SEQ ID NO: 2 with other amino acid residues (Table 2). The resulting expression plasmids containing amino acid substitution mutations were introduced into CHO-K1 cell line using Lipofectamine 2000 (Life Technologies Corp.) to confirm that the recombinant human pancreatic RNase 1 with amino acid substituted mutation having human immunoglobulin kappa chain added to the N-terminal side thereof, is secreted into the medium. The recombinant human pancreatic RNase 1 with amino acid substituted mutation secreted into the medium was used in an experiment to determine antibody specificity.

[Table 2]

| Mutant Name | Amino Acid Mutation introducing Site |
|---|---|
| WT | Mutation-free introducing |
| R85K | Arg85→Lys |
| L86I | Leu86→Ile |
| T87S | Thr87→Ser |
| N88D | Asn88→Asp |
| G89S | Gly89→Ser |
| S90A | Ser90→Ala |
| R91K | Arg91→Lys |
| Y92F | Thy92→Phe |

Measurement of Reactivity of Anti-Human Pancreatic RNase 1 Antibodies to Amino Acid Substituted Mutant Antigens

Reactivity of anti-human pancreatic RNase I antibodies to amino acid substitution mutant antigens was measured by sandwich ELISA as described below. 50 µl of phosphate buffer (50 mM sodium phosphate, 150 mM NaCl, pH 7.4) containing 25 ng of goat anti-human immunoglobulin kappa chain antibody (Sigma-Aldrich Corp.) were added to each well of a 96-well microtiter plate (Greiner Bio-One Co., Ltd.) followed by fixing for 16 hours at 4°C. After washing the wells three times with 300 µl of washing solution (20 mM Tris-HCl, 150 mM NaCl, pH 7.4), 200 µl of TBS solution containing 3% BSA were added followed by allowing to stand for 2 hours at room temperature to carry out blocking (anti-human immunoglobulin kappa chain antibody-immobilized plate). After washing each well three times with 300 µl of washing solution, a culture supernatant of CHO-KI cells containing a plasmid expressing antigen introduced with an amino acid substituted mutation was added followed by allowing to stand for 1 hour at room temperature. After washing each well three times with 300 µl of washing solution containing a surfactant (20 mM Tris-HCl, 150 mM NaCl, 0.05% Tween-20, pH 7.4), 50 µl of a diluted solution containing 25 ng of HRP-labeled mouse anti-human pancreatic RNase 1 antibody (MrhRN0614) or 25 ng of HRP-labeled rat anti-human pancreatic RNase 1 antibody (RrhRN0723) were added followed by allowing to stand for 1 hour at room temperature. Finally, after washing each well three times with 300 µl of washing solution containing surfactant, 50 µl of TMB solution were added followed by coloring for 10 minutes, terminating the reaction by adding 1 M phosphoric acid solution and measuring absorbance at 450 nm.

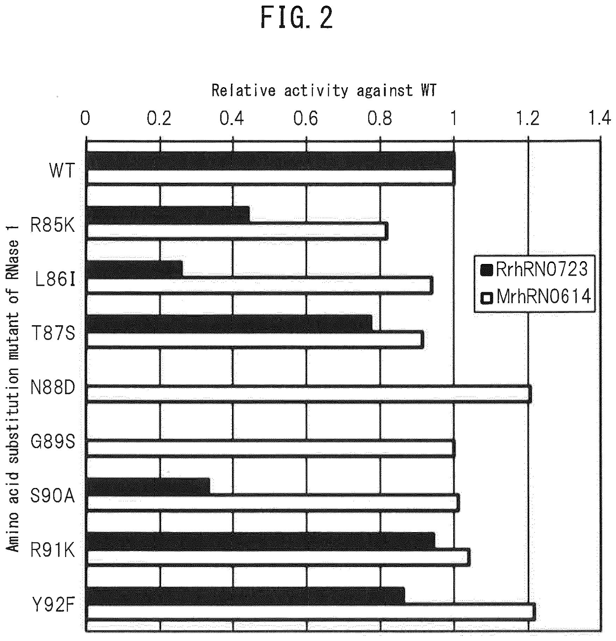

The results of the measurements are shown in FIG. 2. Relative values based on a value of 1.0 for the amount of each antibody bound to the mutation-free recombinant antigen WT are plotted on the horizontal axis. Each of the amino acid substituted mutant antigens are shown on the vertical axis. The horizontal solid black bars indicate the bound amounts of RrhRN0723 antibody, while the horizontal white bars indicate the bound amounts of MrhRN0614 antibody. Since the reactivity of RrhRN0723 antibody to mutants R85K, L86I, N88D, G89S and S90A was much lower than the reactivity of MrhRN0614, anti-human pancreatic RNase 1 antibody RrhRN0723 was confirmed to recognize amino acid residues in the vicinity of the putative glycosylation site in the form of asparagine at position 88, and in particular, from the arginine residue at position 85 to the serine residue at position 90.

Reactivity of Anti-Human Pancreatic RNase 1

Antibody to Antigen with glycan modification at a putative glycosylation

Chain Modifiable Site Asn88

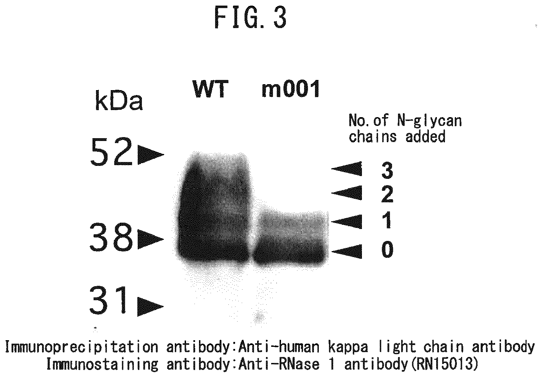

Glycan-modification deficient mutant human pancreatic RNase 1 (m001) expressed in CHO-K1 cells in which only the asparagine residue at position 88 was glycosylated was analyzed by western blotting. Namely, mutation-free recombinant antigen WT and glycan-modification deficient mutant antigen m001 were separated by SDS-PAGE following immunoprecipitation using anti-human immunoglobulin kappa light chain antibody. The separated proteins were transferred to a PVDF membrane and then detected by reacting with anti-human pancreatic RNase 1 antibody (RN15013) that recognizes the 14 amino acid sequence on the N-terminal side of pancreatic RNase 1. Among the expressed recombinant proteins, in the case of WT, molecules ranging from molecules not having a glycan chain added thereto to molecules having glycan chains attached at 1, 2 or 3 locations were detected and the amount of glycan chain added varied, while in the case of m001, two types of molecules consisting of molecules having a glycan chain added at one location and molecules not having a glycan chain added were determined to be contained (FIG. 3).

Next, an examination was made of the reactivity of antibody to glycan-modification deficient mutant antigen m001 in which the asparagine residue at position 88 was subjected to amino acid modification. Namely, glycan-modification deficient mutant antigen m001, in which the asparagine residue at position 88 was subject to glycan chain modification, was fractionated using a lectin column immobilized with concanavalin A lectin that recognizes and binds to N-glycan chains of glycoproteins. More specifically, CHO-K1 cultured cell supernatant in which glycan-modification deficient mutant human pancreatic RNase 1 (m001) had been expressed was subjected into a concanavalin A lectin-conjugated column (HiTrap-ConA, GE Healthcare Inc.) equilibrated with lectin column binding buffer (20 mM Tris-HCl, 150 mM NaCl, 1 mM $CaCl_2$, 0.5 mM $MgCl_2$, pH 7.4) to bind the glycan-modification deficient mutant human pancreatic RNase 1 (m001). After adequately washing the concanavalin A lectin-conjugated column with lectin column binding buffer, the bound glycan-modification deficient mutant human pancreatic RNase 1 (m001) was eluted using lectin column binding buffer containing alpha-methylmannoside (Sigma-Aldrich Corp.). All eluted fractions from the time of sample injection were collected in a fraction collector and the amount of recombinant antigen contained in each of the fractionated fractions was measured by sandwich ELISA. Antibody MrhRN0614, which is not affected by glycan chain modification, and antibody RrhRN0723, which recognizes the vicinity of putative glycosylation site Asn88, were used for detection.

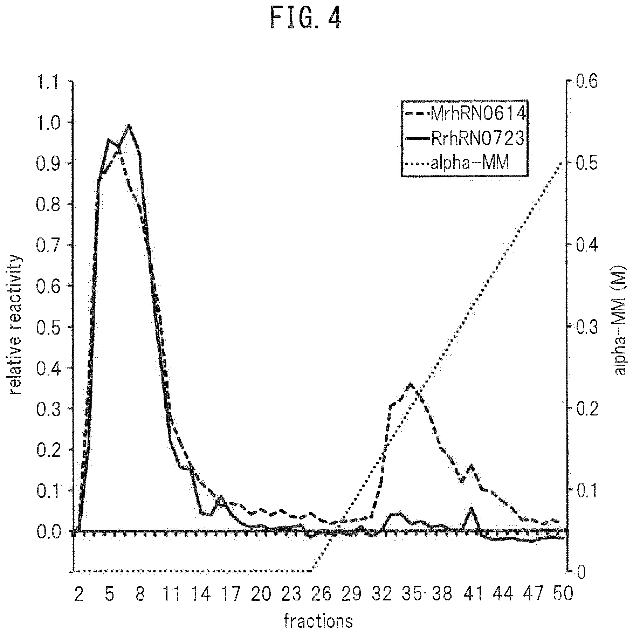

The results are shown in FIG. 4. The fractions obtained by column separation are shown on the horizontal axis, measured values of ELISA analysis are shown on the left vertical axis, and concentrations of alpha-methylmannoside are shown on the right vertical axis. The broken line indicates changes in the bound amount of MrhRN0614 and the solid line indicates in the bound amount of RrhRN0723. The dotted line indicates the concentration of the eluted sugar in the form of alpha-methylmannoside in each fraction. As shown in FIG. 4, although antigen was clearly determined to be eluted in the concanavalin A-bound fraction (alpha-methylmannoside eluted fraction) in the case of MrhRN0614, RrhRN0723 was clearly determined to not demonstrate reactivity to the same fraction. On the other hand, the fraction that did not bind to concanavalin A (flow-through fraction) was clearly determined to demonstrate reactivity with both antibodies. When antigen contained in the concanavalin A-bound fraction was analyzed by western blotting, this fraction was shown to contain only glycosylated antigen. On the basis of these results, the newly acquired anti-human pancreatic RNase 1 antibody (RrhRN0723) was shown to be an antibody that does not react with pancreatic RNase 1 in which the asparagine residue at position 88 has been glycosylated, and only binds in the case that amino acid residue is not glycosylated. Namely, in an immunological assay system using anti-human pancreatic RNase 1 antibody (RrhRN0723), it was determined that the amount of pancreatic RNase 1 is measured in the case a glycan chain is not bound to the asparagine residue at position 88.

Example 3 - Measurement of Human Serum Specimens by Sandwich ELISA

Measurements were carried out by sandwich ELISA using biological samples as specimens.

Measurement of putative N-glycosylation Sites not linked with N-Glycan Chain of Pancreatic RNase 1 in Biological Samples Derived from Pancreatic Cancer Patients

In order to measure those putative N-glycosylation sites of pancreatic RNase 1 without N-glycan chain in human biological samples, samples were prepared consisting of serum from healthy individuals (40 specimens), serum from pancreatic cancer patients (50 specimens, Biotheme Research Solutions, Inc.), serum from pancreatic disease patients (non-cancerous, 12 specimens, Biotheme Research Solutions, Inc.), serum from breast cancer patients (20 specimens, Biotheme Research Solutions, Inc.), serum from benign mammary adenoma (18 specimens), serum from prostate cancer patients (24 specimens, SLR Research Corp.) and serum from prostatomegaly patients (24 specimens, SLR Research Corp.) (Table 3).

[Table 3]

| Specimen Classification | No. of Specimens |
|---|---|
| Serum from healthy individuals | 40 |
| Serum from pancreatic cancer patients | 50 |
| Serum from non-cancerous pancreatic disease patients | 12 |
| Serum from breast cancer patients | 20 |
| Serum from benign mammary adenoma patients | 18 |
| Serum from prostate cancer patients | 24 |
| Serum from prostatomegaly patients | 24 |
| Total | 188 |

Measurements were carried out according to the procedure described below. 50 $\mu$l of phosphate buffer (50 mM sodium phosphate, 150 mM NaCl, pH 7.4) containing 25 ng of mouse anti-human pancreatic RNase 1 antibody (MrhRN0614) were added to each well of a 96-well microtiter plate (Greiner Bio-One Co., Ltd.) followed by fixing for 16 hours at 4°C. After washing the wells three times with 300 $\mu$l of washing solution (20 mM Tris-HCl, 150 mM NaCl, pH 7.4), 200 $\mu$l of blocking solution containing 3% BSA (3% BSA, 20 mM Tris-HCl, 150 mM NaCl, pH 7.4) were added followed by allowing to stand for 2 hours at room temperature to carry out blocking (anti-human pancreatic RNase 1 antibody-immobilized plate). After washing each well three times with 300 $\mu$l of washing solution, pancreatic cancer patient serum or healthy individual serum used as a control specimen, diluted 10-fold with a diluent (1% BSA, 20 mM Tris-HCl, 150 mM NaCl, 0.05% Tween-20, pH 7.4), was added followed by allowing to stand for 1 hour at room temperature. After washing each well three times with 300 $\mu$l of washing solution containing a surfactant (20 mM Tris-HCl, 150 mM NaCl, 0.05% Tween-20, pH 7.4), 50 $\mu$l of a diluted solution containing 25 ng of HRP-labeled rat anti-human pancreatic RNase 1 antibody (RrhRN0723) were added followed by allowing to stand for 1 hour at room temperature. Next, after washing each well three times with 300 $\mu$l of washing solution, 50 $\mu$l of TMB solution were added followed by coloring for 10 minutes, terminating the reaction by adding 1 M phosphoric acid solution and measuring absorbance at 450 nm. Other specimens were measured in the same manner.

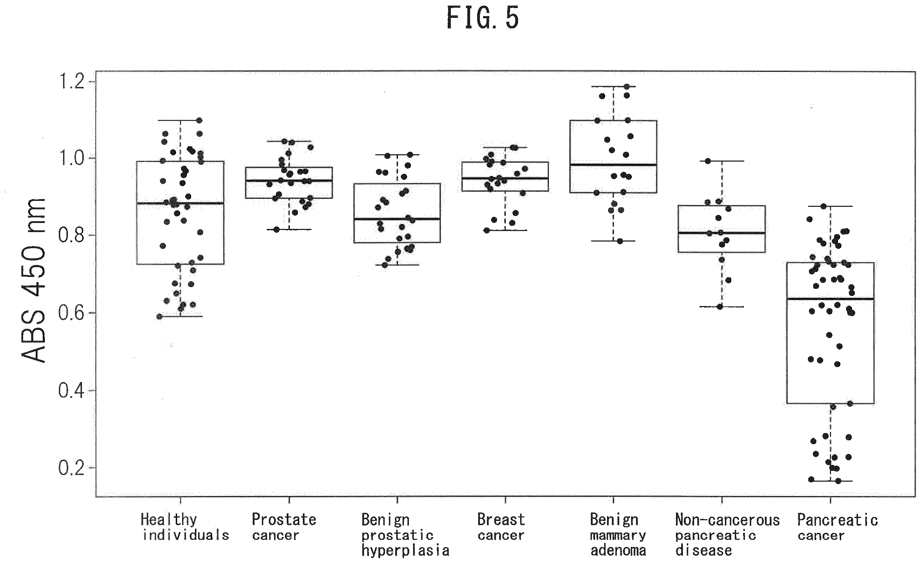

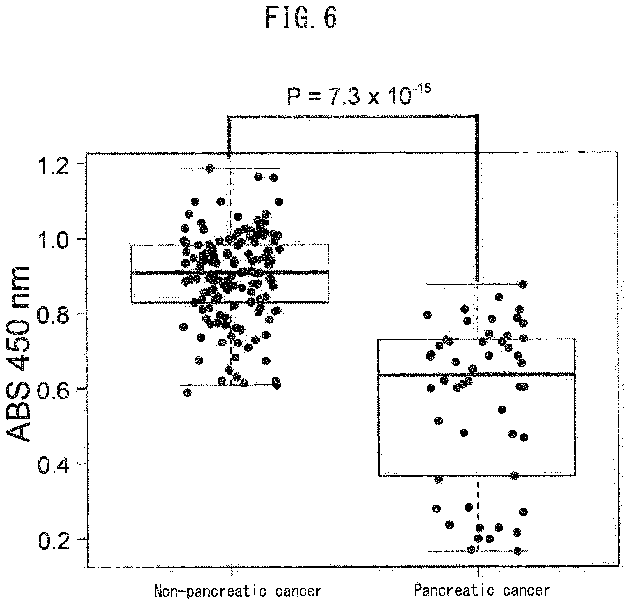

The results are shown in FIG. 5. The types of cancer are plotted on the horizontal axis and measured values obtained from ELISA assay are plotted on the vertical axis. The black dots indicate the measured value of each specimen and the statistical distributions thereof are indicated with the boxes. In contrast to diseases other than pancreatic cancer demonstrating a certain specific value or higher, pancreatic cancer exhibited significantly lower values in comparison with the other groups. Namely, in the case of pancreatic cancer patients, there were indicated to be few cases in which an N-glycan chain is not linked to the putative N-glycosylation site of pancreatic RNase 1 recognized by RrhRN0723 in comparison with healthy individuals. Conversely, in the case of pancreatic cancer patients, there were indicated to be many cases in which N-glycan chain is bound to this putative N-glycosylation site in comparison with healthy individuals. In addition, as a result of testing for a significant difference in the case of dividing into a group obtained by combining the six groups other than pancreatic cancer (non-pancreatic cancer group) and a pancreatic cancer group, the resulting p value was extremely low at $7.3 \times 10^{-15}$, thereby clearly demonstrating the presence of a significant difference (FIG. 6).

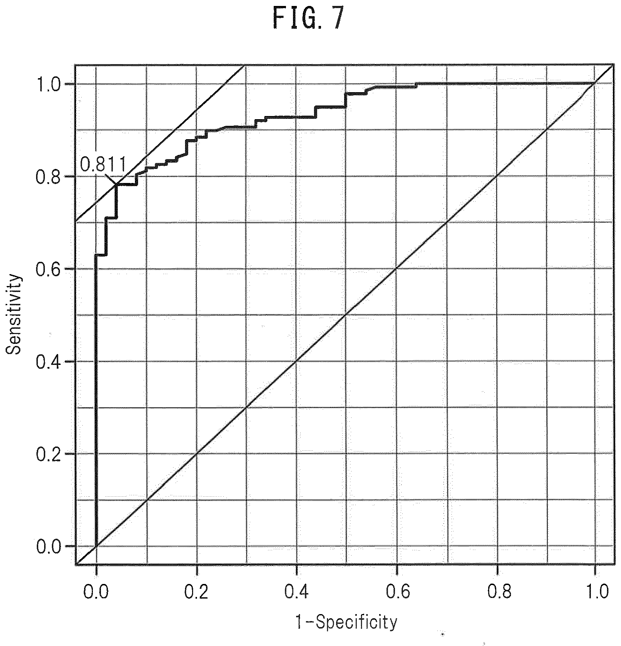

On the basis of these measurement results, the results of analysis of discrimination accuracy for pancreatic cancer are shown in FIG. 7 and Table 4. An ROC statistical analysis was carried out using a statistical analysis software R. Specificity at the optimal cutoff value (ABS 450 nm = 0.811) was 96% and sensitivity was 78%, and AUC, which is used as an indicator of diagnostic performance, was sufficiently high at 0.933.

[Table 4]

| Specificity | 96% |
|---|---|
| Sensitivity | 78% |
| AUC | 0.933 |

Example 4 - Examination of Glycan Chain Binding at Putative Glycosylation Site Asn88 of Pancreatic RNase 1 in Pancreatic Cancer Patient Serum

An antibody that recognizes and binds to the putative glycosylation site Asn88 of pancreatic RNase 1 was produced in accordance with the following procedure and used as primary antibody in western blotting.

Immunization of Immune Animals

A peptide obtained by chemically synthesizing a portion equivalent to amino acid sequence 85 to 95 of SEQ ID NO: 2 (SEQ ID NO: 4) was conjugated with KLH and used as an immunogen, and this was used to immunize rats. More specifically, 100 μg of immunogen were administered into the foot pads of both back legs of 6-week-old WHY female rats together with Freund's complete adjuvant for the purpose of initial immunization. Subsequently, 100 μg of immunogen were administered into the foot pads of the back legs 28 days later together with physiological saline for the purpose of final immunization.

Production of Antibody-Producing Hybridomas

Iliac lymph nodes and inguinal lymph nodes were excised from the immunized rats 3 days after final immunization to obtain lymph node cells. After fusing the rat lymph node cells to a mouse myeloma cell line by electrofusion, hybridomas were selected by inoculating onto a 96-well cell culture plate using GIT medium (Wako Pure Chemical Industries Co., Ltd.) containing hypoxanthine, aminopterin and thymidine.

Selection of Hybridoma Line Producing Rat Anti-Human Pancreatic RNase 1 Antibody

A hybridoma line producing rat anti-human pancreatic RNase 1 peptide antibody was selected by carrying out screening of an antibody from antibodies secreted into the medium by the aforementioned hybridomas with the ELISA method using a screening antigen which is the BSA conjugated with the peptides for screening (SEQ ID NO: 5 or 6). The ELISA procedure used for screening was as indicated below. 50 μl of phosphate buffer (50 mM sodium phosphate, 150 mM NaCl, pH 7.4) containing 25 ng of screening peptide-conjugated BSA were respectively added to each well of a 96-well microtiter plate (Greiner Bio-One Co., Ltd.) followed by fixing for 16 hours at 4°C. After washing the wells three times with 300 μl of washing solution (20 mM Tris-HCl, 150 mM NaCl, pH 7.4), 200 μl of blocking solution containing 3% BSA (3% BSA, 20 mM Tris-HCl, 150 mM NaCl, pH 7.4) were added followed by allowing to stand for 2 hours at room temperature to carry out blocking (screening peptide-conjugated BSA-immobilized plate). After washing each well three times with 300 μl of washing solution, 50 μl of hybridoma culture supernatant were respectively added to each well followed by allowing to stand for 1 hour at room temperature. Next, after washing each well three times with 300 μl of washing solution containing a surfactant, 50 μl of a diluted solution containing 0.01 μg of HRP-labeled anti-rat IgG antibody (American Qualex Scientific Products) were added followed by allowing to stand for 1 hour at room temperature. Finally, after washing each well three times with 300 μl of washing solution containing surfactant, 50 μl of TMB solution were added followed by coloring for 15 minutes. Subsequently, the reaction was terminated by adding 1 M phosphoric acid solution followed by measurement of absorbance at 450 nm. The criterion for selection of a hybridoma line producing anti-human pancreatic RNase 1 peptide antibody among the aforementioned screening peptides consisted of hybridomas that produce antibody that reacts to the peptide of SEQ ID NO: 5 but does not react to the peptide of SEQ ID NO: 6. Furthermore, the peptide of SEQ ID NO: 5 contains a sequence equivalent to the amino acids at positions 85 to 95 of naturally-occurring pancreatic RNase 1 (SEQ ID NO: 2). In addition, the peptide of SEQ ID NO: 6 is equivalent to a sequence in which the Asn residue at position 5 in the peptide of SEQ ID NO: 5 is substituted with an Asp residue, and the substitution site is equivalent to the Asn residue at position 88 of SEQ ID NO: 2.

A supernatant of the selected hybridoma line was further screened by western blotting, and a hybridoma line that produces antibody which was able to be confirmed to bind to mutation-free recombinant antigen WT on a PVDF membrane of the western blot was ultimately selected for the hybridoma line.

Based on the results of screening, a fused cell line was obtained that produces an antibody capable of specifically detecting pancreatic RNase 1 as a primary antibody of western blotting. The resulting fused cells were subjected to limiting dilution to obtain monoclonal antibody RN3F34.

Confirmation of Antigen Recognition Specificity of Rat Anti-Human Pancreatic RNase 1 Peptide Antibody

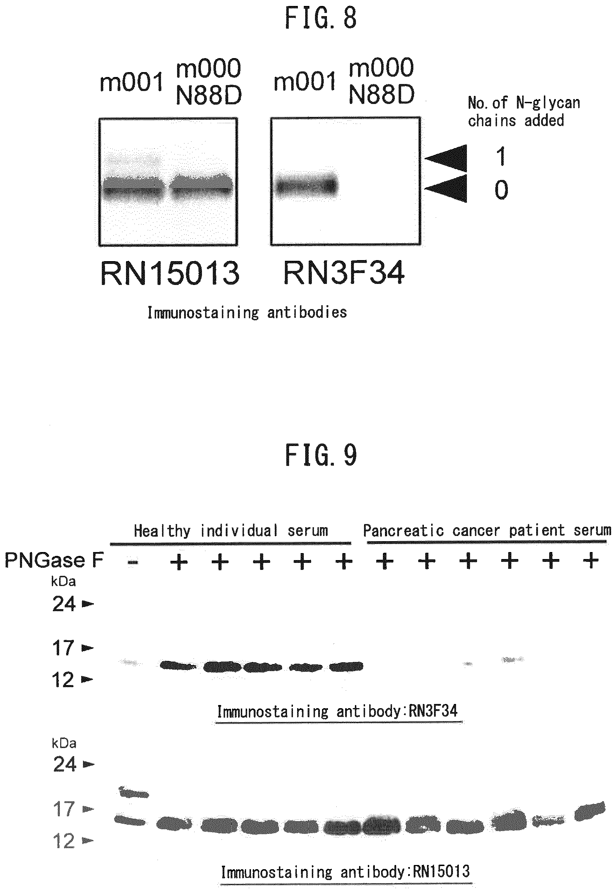

In order to confirm the antigen recognition specificity of the resulting rat anti-human pancreatic RNase 1 peptide antibody (RN3F34), the reactivity to glycan-modification deficient mutant human pancreatic RNase 1 in which only the putative glycosylation site of Asn88 remained (m001) was confirmed by western blotting. As shown in FIG. 8, in the case of antibody RN15013 that binds to the 14 amino acid sequence on the N-terminal side of pancreatic RNase 1, both glycosylated m001 molecules on the high molecular weight and molecules without glycosylation on the low molecular weight side were detected. In contrast, in the case of RN3F34 antibody, only molecules without glycosylation on the low molecular weight side were detected. On the basis of the above results, the resulting rat anti-human pancreatic RNase 1 peptide antibody (RN3F34) was confirmed to be an antibody that does not bind to pancreatic RNase 1 having a structure of glycan chain at Asn88.

A mutant expression vector in which Asn at position 88 of mutant m001 was substituted with aspartic acid (pcDNA-KFH-hRNaseI m000-N88D) was produced by PCR mutagenesis using the aforementioned expression vector (pcDNA-KFH-hRHaseI m001) as a template to similarly confirm the reactivity to mutant-introduced antigen (m000-N88D, Table 1) expressed in cell line CHO-K1. As shown in FIG. 8, in contrast to m000-N88D having been detected by antibody RN15013 that binds to the 14 amino acid sequence on the N-terminal side of pancreatic RNase 1, rat anti-human pancreatic RNase 1 peptide antibody (RN3F34) was able to be confirmed to not be able to detect m000-N88D. In this manner, that in which Asn88 was substituted with aspartic acid was confirmed to not be bound by RN3R34 antibody. Namely, this means that, in the case of having carried out deglycosylation treatment of a glycan chain linked to Asn88 with PNGaseF and the like, RN3F34 antibody does not bind to the pancreatic RNase 1 in which asparagine has been converted to aspartic acid accompanying that deglycosylation treatment. Thus, rat anti-human pancreatic RNase 1 peptide antibody (RN3F34) does not bind to a site which is the Asn88 of pancreatic RNase 1 with glycan chain and was subsequently converted to aspartic acid by deglycosylation treatment with PNGaseF, but does bind to a site which does not undergo amino acid substitution by deglycosylation treatment due to the Asn88 of pancreatic RNase 1 was not glycosylated. In this manner, in the case of observing reactivity following deglycosylation treatment with PNGaseF and the like, RNF34 antibody demonstrates performance comparable to that of RrhRN0723 in the sense that it binds in the case a glycan chain is not bound at Asn at position 88 of the amino acid sequence of SEQ ID NO: 2, but does not bind in case a glycan chain is bound at that site.

Confirmation of Change in Glycan Chain linked to Asn88 of Pancreatic RNase 1 in Human Serum

The presence or absence of glycan chain binding at Asn88 in pancreatic RNase 1 in human serum was determined by detecting pancreatic RNase 1 in human serum that underwent deglycosylation treatment with PNGaseF followed by western blotting using rat anti-human pancreatic RNase 1 peptide antibody (RN3F34). Namely, pancreatic RNase 1 was extracted from 5 specimens of healthy individual serum and 6 specimens of pancreatic cancer patient serum by immunoprecipitation using MrhRN0614 antibody, and after denaturing the pancreatic RNase 1 with solubilizing denaturation buffer, deglycosylation treatment was carried out with PNGaseF. The treated samples were analyzed by western blotting. RN15013, which recognize the 14 amino acid sequence on the N-terminal side of pancreatic RNase 1, and anti-human pancreatic RNase 1 peptide antibody RN3F34 were used for detection. As shown in FIG. 9, in the case of RN15013, constant amounts of pancreatic RNase 1 were detected in both the healthy individual serum and pancreatic cancer patient serum, and there were no remarkable differences observed between the two. On the other hand, in the case of detection with RN3F34, in contrast to pancreatic RNase 1 having been detected in healthy individual serum, it was not detected in pancreatic cancer patient serum or the amount detected was remarkably lower than that in healthy individual serum. On the basis of this result, it was confirmed that the pancreatic RNase 1 in the serum of many case of pancreatic cancer patients has an Asn88 linked with an N-glycan chain in comparison with in serum of healthy individuals.

Example 5 - Detection of Pancreatic Cancer by Determining Ratio of Amount of the Putative Glycosylation Site of Asn88 not linked with glycan to the Amount of the Putative Glycosylation Site of Asn88

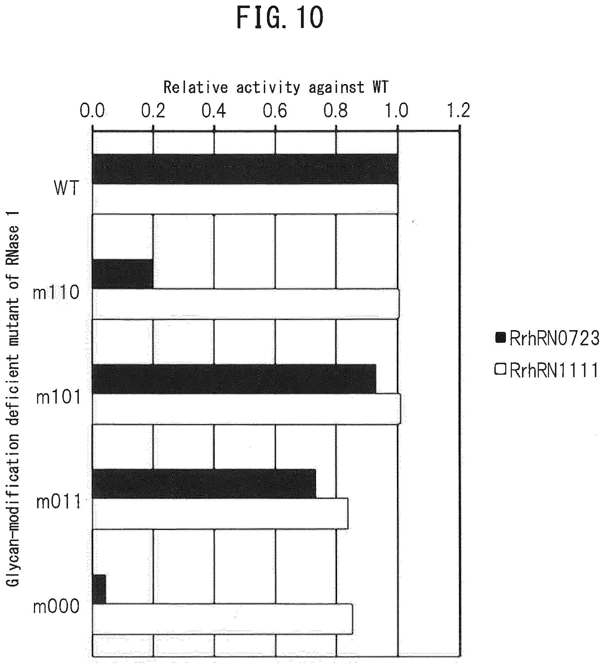

An immunoassay reagent that uses two antibodies that specifically recognize human pancreatic RNase 1 and have a capability of binding to it regardless of the presence or absence of glycan chain at its three putative glycosylation sites, and an immunoassay reagent that specifically measures RNase 1 in which a glycan chain is not linked to ASn88 were produced, and RNase 1 in serum samples was measured with each of these reagents. More specifically, the previously described antibodies MrhRN0614 and RrhRN1111 were used for the two antibodies that specifically recognize human pancreatic RNase 1 and have a capability of binding to it regardless of the presence or absence of glycan chain at its three putative glycosylation sites. RrhRN1111 was isolated during acquisition of RrhRN0723 as an antibody capable of binding to pancreatic RNase 1 regardless of the presence or absence of glycan chain at three putative glycosylation sites. The antigen recognition specificity of RrhRN1111 was examined using the same method as ELISA using a glycan-modification deficient mutant antigen that was used to measure antibody specificity in Example 2. The results are shown in FIG. 10. Relative values based on a value of 1.0 for the amount of each antibody that binds to mutation-free recombinant antigen WT are plotted on the horizontal axis. Each glycan-modification deficient mutant antigen is plotted on the vertical axis. The horizontal solid black bars indicate the bound amounts of RrhRN0723 antibody, while the horizontal white bars indicate the bound amounts of RrhRN1111 antibody. As shown in FIG. 10, in contrast to RrhRN0723 not reacting to mutant m000 that contains mutations at all of putative glycosylation sites and does not undergo any glycan chain modification, reactivity of RrhRN1111 to m000 maintained a level of about 90% in comparison with mutation-free recombinant antigen WT. Accordingly, RrhRN1111 was confirmed to be able to bind to pancreatic RNase 1 regardless of the presence or absence of glycan chain at the three putative glycosylation sites.

The following indicates methods for preparing and evaluating assay reagents of an immunoassay system developed using these antibodies. MrhRN0614 was physically adsorbed to a water-insoluble carrier (made of EVA and incorporating particles having a particle diameter of about 1.5 mm kneaded with ferrite) at 90 ng/carrier, and blocking treatment was carried out using BSA following adsorption. The water-insoluble carrier is able to physically adsorb about 100 ng of protein per carrier. After placing 12 carriers in a magnetically permeable container (volume: 1.2 ml), a buffer containing 0.5 $\mu$g/ml of alkaline phosphatase-labeled RrhRN0723 or 0.5 $\mu$g/ml of alkaline phosphatase-labeled RrhRN1111 (1% BSA, 2.5% dextran, 150 mM NaCl, 0.05% Tween-20, 20 mM Tris buffer, pH 7.4) was added followed by freeze-drying. This reagent was measured by fully automated measurement using a commercially available fully automated immunoassay system (Tosoh Corp., trade name: AIA-600II). The measurement principle is as indicated below. Namely, 150 $\mu$L of measurement sample were added followed by moving the water-insoluble carrier using a magnet for 10 minutes at 37°C and carrying out an immune reaction while stirring the mixture. Following the reaction, a B/F separation procedure was carried out to separate and remove the free labeled antibody followed by adding an alkaline phosphatase substrate in the form of 4-methylumbelliferyl phosphate and measuring the formation rate per unit time (nM/sec) of the enzyme reaction degradation product (4-methylumbelliferone) for 20 seconds to 295 seconds after adding the substrate. Samples were measured using the aforementioned freeze-dried reagent containing labeled antibody RrhRN0723 followed by determination of the amount of the putative glycosylation site at position 88 of pancreatic RNase 1 linked with a glycan chain (to be referred to as the $F_3$ value). In addition, samples were measured using the aforementioned freeze-dried reagent containing labeled antibody RrhRnlll followed by determining the amount of pancreatic RNase 1 (to be referred to as the total (t) value). The ratio thereof ($F_3$/t) was determined according to the equation indicated below.

$$F_3/t = F_3 \text{ value/total value} \qquad (1)$$

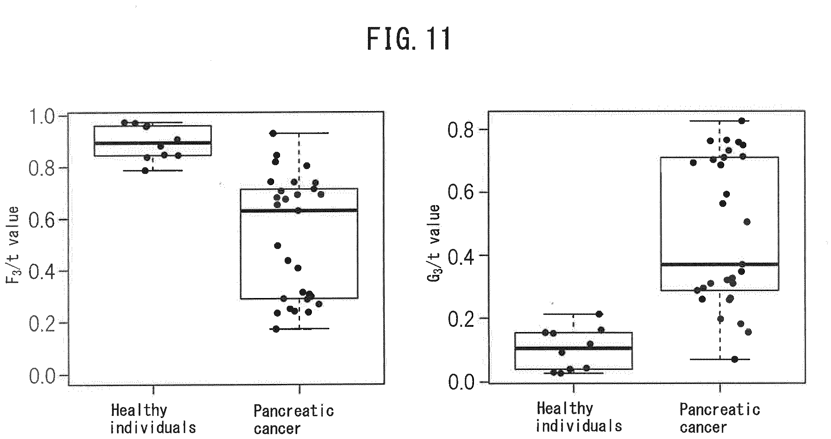

The results are shown in FIG. 11. In contrast to the $F_3$/t value in serum specimens obtained from healthy individuals being about 0.8 to 1.0, $F_3$/t values of specimens obtained from pancreatic cancer patients were widely distributed over a range of about 0.1 to 0.7, and were confirmed to be significantly different from the values for healthy individual specimens.

The ratio of the amount of putative glycosylation site at position 88 with N-glycan to the total value of pancreatic RNase 1 ($G_3$/t) was determined by determining the value of equation (2) below. Those results are shown in FIG. 11. The ratios of specimens obtained from pancreatic cancer patients were confirmed to be significantly different from those of specimens obtained from healthy individuals.

$$G_3/t = 1 - (F_3/t) \qquad (2)$$

Example 6 - Measurement by Antigen Immobilized Competition Method

A method was developed for measuring the amount of the Asn88 putative glycosylation site of pancreatic RNase 1 where glycan chain is not bound by an antigen immobilize competition method. Namely, RNase 1 that reacts to RrhRN0723 was purified from a culture supernatant of pancreatic cancer-derived cultured cell line Capan-1 using a RrhRN0723-immobilized column. After concentrating this antigen, the concentration of protein in the antigen solution was determined with a protein assay kit. The purified RNase 1 was biotinated using a biotin introducing reagent (sulfoNHS-LCLC-biotin, Thermo Scientific Inc.) to obtain a solid phase antigen. About 2.5 $\mu$g of the aforementioned RNase 1 was immobilized in 100 $\mu$l of a suspension of streptavidin-immobilized Dynabeads (Dynal Corp.) to produce antigen-immobilized beads.

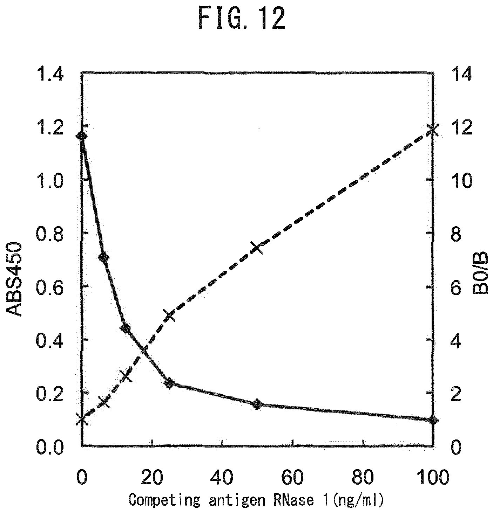

These antigen-immobilized beads (4 $\mu$l), HRP-labeled RrhRN0723 antibody (10 ng) and serially diluted competing antigen (0 ng to 100 ng) were mixed followed by allowing to react for 1 hour. Following the reaction, after recovering only the magnetic beads from the solution using a magnet and washing three times with washing solution (20 mM Tris-HCl, 150 mM NaCl, 0.05% Tween-20, pH 7.4), TMB solution was added and the beads were colored for 10 minutes. The reaction was terminated by adding 1 M phosphoric acid to the colored solution followed by measuring absorbance at 450 nm to measure the amount of HRP-labeled RrhRN0723 antibody bound to the beads. FIG. 12 shows the results of measuring the aforementioned purified RNase 1 by an antigen immobilized competition method. The solid line indicates measured values (first axis) while the broken line (second axis) indicates values obtained by dividing measured values when antigen concentration is 0 by the measured values at each concentration. Measured values can be seen to change dependent on the amount of RNase 1 introduced into the reaction as a competing antigen. On the basis of the above results, the amount of RNase 1 without N-glycan chain at Asn88 was shown to be able to be measured by an antigen immobilized competition method using RrhRN0723 antibody.

## Claims

1. A method for detecting pancreatic cancer in a human subject, **characterized by** measuring the amount of either a putative N-glycosylation site of the pancreatic ribonuclease 1 linked with an N-glycan chain or not linked with an N-glycan chain, wherein the putative N-glycosylation site is one or more asparagine residues selected from asparagine at positions 34, 76 and 88 of SEQ ID NO: 2, and wherein the amount of the site linked with the N-glycan chain is increased in pancreatic cancer patients in comparison with healthy individuals and the amount of the site not linked with the N-glycan chain is decreased in pancreatic cancer patients in comparison with healthy individuals.

2. A method for detecting pancreatic cancer in a human subject, comprising: determining the ratio A/B for A and B indicated below:

    A: amount of putative N-glycosylation site of either the pancreatic ribonuclease 1 linked with an N-glycan chain or not linked with an N-glycan chain; and,
    B: amount of putative N-glycosylation site of the pancreatic ribonuclease 1,

    wherein the putative N-glycosylation site is one or more asparagine residues selected from asparagine at positions 34, 76 and 88 of SEQ ID NO: 2; and wherein
    when A represents the amount of the site not linked with an N-glycan chain, the value of A/B is lower in pancreatic cancer patients in comparison with healthy individuals; and
    when A represents the amount of the site linked with an N-glycan chain, the value of A/B is higher in pancreatic cancer patients in comparison with healthy individuals.

3. The method according to any of claims 1 to 2, wherein the putative N-glycosylation site is an asparagine residue at position 88 of SEQ ID NO: 2.

4. The method according to any of claims 1 to 2, wherein the putative N-glycosylation site is an asparagine residue at position 76 of SEQ ID NO: 2.

5. The method according to any of claims 1 to 2, wherein the putative N-glycosylation site is an asparagine residue at position 34 of SEQ ID NO: 2.

6. A monoclonal antibody, or fragment thereof, both of which recognize a putative N-glycosylation site of the antigen recognition site of human pancreatic RNase 1, wherein the putative N-glycosylation site is one asparagine residue selected from asparagine at positions 34, 76 and 88 of SEQ ID NO: 2, and wherein binding to the putative N-glycosylation site of human pancreatic RNase 1 occurs in the case that the site is not linked with an N-glycan chain, and is statistically significantly inhibited in the case that the site is linked with an N-glycan chain.

7. The monoclonal antibody, of fragment thereof, according to claim 6, wherein the antigen recognition site contains the putative N-glycosylation site being the asparagine residue at position 88 of SEQ ID NO: 2.

8. A monoclonal antibody or fragment thereof, both of which are capable of binding to human pancreatic RNase 1 simultaneously with the binding to human pancreatic RNase 1 of the monoclonal antibody, or fragment thereof, according to any of claims 6 to 7.

9. The method according to any of claims 1 to 5, comprising contacting the monoclonal antibody or fragment thereof according to claim 6 or 7 with a sample, and measuring the amount of pancreatic RNase 1 that has formed a complex with said monoclonal antibody or a fragment thereof.

10. The method according to claim 9, comprising further contacting the monoclonal antibody or fragment thereof according to claim 8 with the sample previously contacted by the monoclonal antibody or fragment thereof according to claim 6 or 7, and measuring the amount of pancreatic RNase 1 that has formed a complex with said two monoclonal antibodies or fragments thereof.

11. The method according to claim 9 or 10, comprising a first step to contact the sample with the monoclonal antibody or fragment thereof according to claim 6 or 7 or with the monoclonal antibody or fragment thereof according to claim 8, and a second step to further contact the sample obtained in the first step, respectively, with the monoclonal antibody or fragment thereof according to claim 8 or with the monoclonal antibody or fragment thereof according to claim 6 or 7.

12. A pharmaceutical containing the monoclonal antibody, or fragment thereof, according to any of claims 6 to 7.

13. The method according to any of claims 1 to 5, wherein the amount of the putative N-glycosylation site of the pancreatic ribonuclease 1 linked with N-glycan chain or not linked with the N-glycan chain is determined by mass spectrometry.

14. The method according to claim 13, wherein determining by mass spectrometry includes decomposing the pancreatic ribonuclease 1 with an enzyme and then measuring the mass of the resulting peptide fragment and/or glycopeptide fragment.

**Patentansprüche**

1. Verfahren für den Nachweis von Bauchspeicheldrüsenkrebs bei einem Menschen, **gekennzeichnet durch** ein Messen der Menge entweder einer mutmaßlichen N-Glycosylierungsstelle der Pankreas-Ribonuklease 1, die mit einer N-Glycankette verbunden ist oder die nicht mit einer N-Glycankette verbunden ist, wobei die mutmaßliche N-Glykosylierungsstelle ein Asparaginrest ist oder mehrere Asparaginreste sind, die aus einem Asparagin an den Positionen 34, 76 und 88 der SEQ ID NO: 2 ausgewählt worden sind, und wobei die Menge der Stelle, die mit der N-Glycankette verbunden ist, bei Patienten mit Bauchspeicheldrüsenkrebs im Vergleich mit gesunden Individuen erhöht ist und wobei die Menge der Stelle, die nicht mit der N-Glycankette verbunden ist, bei Patienten mit Bauchspeicheldrüsenkrebs im Vergleich mit gesunden Individuen vermindert ist.

2. Verfahren für den Nachweis von Bauchspeicheldrüsenkrebs bei einem Menschen, welches aufweist: ein Bestimmen des Verhältnisses von A/B für ein A und ein B, wie unten angegeben:

   A: Menge der mutmaßlichen N-Glycosylierungsstelle von entweder der Pankreas-Ribonuclease 1, die mit einer N-Glycankette verbunden ist, oder der, die nicht mit einer N-Glycankette verbunden ist, und
   B: Menge der mutmaßlichen N-Glykosylierungsstelle der Pankreas-Ribonuklease 1,

   wobei die mutmaßliche N-Glycosylierungsstelle ein Asparaginrest ist oder mehrere Asparaginreste sind, die aus dem Asparagin an den Positionen 34, 76 und 88 der SEQ ID NO: 2 ausgewählt worden sind, und wobei
   wenn das A die Menge der Stelle darstellt, die nicht mit einer N-Glycankette verbunden ist, der Wert von A/B bei Patienten mit Bauchspeicheldrüsenkrebs im Vergleich mit gesunden Individuen niedriger ist, und
   wenn das A die Menge der Stelle darstellt, die mit einer N-Glycankette verbunden ist, der Wert von A/B bei Patienten mit Bauchspeicheldrüsenkrebs im Vergleich mit gesunden Individuen höher ist.

3. Verfahren nach einem der Ansprüche 1 bis 2, wobei die mutmaßliche N-Glycosylierungsstelle ein Asparaginrest an der Position 88 der SEQ ID NO: 2 ist.

4. Verfahren nach einem der Ansprüche 1 bis 2, wobei die mutmaßliche N-Glycosylierungsstelle ein Asparaginrest an der Position 76 der SEQ ID NO: 2 ist.

**5.** Verfahren nach einem der Ansprüche 1 bis 2, wobei die mutmaßliche N-Glycosylierungsstelle ein Asparaginrest an der Position 34 der SEQ ID NO: 2 ist.

**6.** Monoklonaler Antikörper oder ein Fragment davon, die beide eine mutmaßliche N-Glykosylierungsstelle der Antigenerkennungsstelle der humanen pankreatischen RNase 1 erkennen, wobei die mutmaßliche N-Glykosylierungsstelle ein Asparaginrest ist, das aus einem Asparagin an den Positionen 34, 76 und 88 der SEQ ID NO: 2 ausgewählt worden ist, und wobei ein Binden an die mutmaßliche N-Glykosylierungsstelle von menschlicher Pankreas-RNase 1 in dem Fall auftritt, dass die Stelle nicht mit einer N-Glycankette verbunden ist, und statistisch signifikant inhibiert wird, wenn die Stelle mit einer N-Glycankette verbunden ist.

**7.** Monoklonaler Antikörper oder ein Fragment davon nach Anspruch 6, wobei die Antigenerkennungsstelle die mutmaßliche N-Glykosylierungsstelle enthält, die der Asparaginrest an der Position 88 der SEQ ID Nr. 2 ist.

**8.** Monoklonaler Antikörper oder ein Fragment davon, die beide in der Lage sind, gleichzeitig mit der Bindung des monoklonalen Antikörpers des Pankreas-RNase 1 oder eines Fragments davon nach einem der Ansprüche 6 bis 7, an die menschliche Pankreas-RNase 1 zu binden.

**9.** Verfahren nach einem der Ansprüche 1 bis 5, welches ein In-Kontakt-Bringen des monoklonalen Antikörpers oder eines Fragments davon nach Anspruch 6 oder 7 mit einer Probe und das Messen der Menge an Pankreas-RNase 1 aufweist, die einen Komplex mit dem monoklonalen Antikörper oder dem Fragment davon gebildet hat.

**10.** Verfahren nach Anspruch 9, welches ferner ein In-Kontakt-Bringen des monoklonalen Antikörpers oder des Fragments davon nach Anspruch 8 mit der Probe, die zuvor durch den monoklonalen Antikörper oder das Fragment davon nach Anspruch 6 oder 7 in Kontakt gebracht worden ist, und welches ein Messen der Menge der pankreatischen RNase 1 aufweist, die einen Komplex mit den zwei monoklonalen Antikörpern oder deren Fragmenten gebildet hat.

**11.** Verfahren nach Anspruch 9 oder 10, welches einen ersten Schritt zum In-Kontakt-Bringen der Probe mit dem monoklonalen Antikörper oder eines Fragment davon nach Anspruch 6 oder 7 oder mit dem monoklonalen Antikörper oder einem Fragment davon nach Anspruch 8 und einen zweiten Schritt zum weiteren In-Kontakt-Bringen der Probe aufweist, die in der ersten Stufe mit dem monoklonalen Antikörper oder einem Fragment davon nach Anspruch 8 erhalten worden ist oder die mit dem monoklonalen Antikörper oder einem Fragment davon nach Anspruch 6 oder 7 erhalten worden ist.

**12.** Pharmazeutikum, das den monoklonalen Antikörper oder das Fragment davon nach den Ansprüchen 6 bis 7 enthält.

**13.** Verfahren nach einem der Ansprüche 1 bis 5, wobei die Menge der mutmaßlichen N-Glycosylierungsstelle der Pankreas-Ribonuclease 1, die mit der N-Glycankette verbunden ist oder die nicht mit der N-Glycankette verbunden ist, durch eine Massenspektrometrie bestimmt wird.

**14.** Verfahren nach Anspruch 13, wobei das Bestimmen durch die Massenspektrometrie ein Zersetzen der Pankreas-Ribonuklease 1 mit einem Enzym und dann ein Messen der Masse des sich daraus ergebenden Peptidfragments und / oder des sich daraus ergebenden Glycopeptidfragments umfasst.

**Revendications**

**1.** Procédé pour la détection d'un cancer du pancréas chez un sujet humain, **caractérisé par** la mesure de la quantité d'un site de N-glycosylation potentiel de la ribonucléase 1 pancréatique lié à une chaine N-glycane ou non lié à une chaine glycane, dans lequel le site de N-glycosylation potentiel est un ou plusieurs résidus asparagine choisis parmi l'asparagine aux positions 34, 76 et 88 de SEQ ID NO : 2, et dans lequel la quantité de site lié à la chaine N-glycane est augmentée chez des patients atteints d'un cancer du pancréas par rapport à des sujets sains et la quantité de site non lié à la chaine glycane est diminuée chez des patients atteints d'un cancer du pancréas par rapport à des sujets sains.

**2.** Procédé pour la détection d'un cancer du pancréas chez un sujet humain, comprenant la détermination du ratio A/B pour A et B indiqués ci-dessous :

A : quantité de site de N-glycosylation potentiel de la ribonucléase 1 pancréatique lié à une chaine N-glycane ou non lié à une chaine N-glycane ; et,

B : quantité de site de N-glycosylation potentiel de la ribonucléase 1,

dans lequel le site de N-glycosylation potentiel est un ou plusieurs résidus asparagine choisis parmi l'asparagine aux positions 34, 76 et 88 de SEQ ID NO : 2, et dans lequel lorsque A représente la quantité de site non lié à une chaine N-glycane, la valeur A/B est inférieure chez les patients atteints d'un cancer du pancréas par rapport à des sujets sains ; et

lorsque A représente la quantité de site lié à une chaine N-glycane, la valeur A/B est plus élevée chez les patients atteints d'un cancer du pancréas par rapport à des sujets sains.

3. Procédé selon l'une quelconque des revendications 1 ou 2, dans lequel le site de N-glycosylation potentiel est un résidu asparagine à la position 88 de SEQ ID NO : 2.

4. Procédé selon l'une quelconque des revendications 1 ou 2, dans lequel le site de N-glycosylation potentiel est un résidu asparagine à la position 76 de SEQ ID NO : 2.

5. Procédé selon l'une quelconque des revendications 1 ou 2, dans lequel le site de N-glycosylation potentiel est un résidu asparagine à la position 34 de SEQ ID NO : 2.

6. Anticorps monoclonal, ou fragment de celui-ci, qui reconnaissent tous deux un site de N-glycosylation potentiel du site de reconnaissance de l'antigène de la RNase 1 pancréatique humaine, dans lequel le site de N-glycosylation potentiel est un résidu asparagine choisi parmi l'asparagine aux positions 34, 76 et 88 de SEQ ID NO : , et dans lequel la liaison au site de N-glycosylation potentiel de la RNase 1 pancréatique humaine se produit dans le cas où le site n'est pas lié à une chaine N-glycane, et est statistiquement significativement inhibé dans le cas où le site est lié à une chaine N-glycane.

7. Anticorps monoclonal, ou fragment de celui-ci, selon la revendication 6, dans lequel le site de reconnaissance de l'antigène contient le site de N-glycosylation potentiel qui est le résidu asparagine à la position 88 de SEQ ID NO : 2.

8. Anticorps monoclonal, ou fragment de celui-ci, qui sont capables tous deux de se lier à la RNase 1 pancréatique humaine simultanément avec la liaison à la RNase 1 pancréatique humaine de l'anticorps monoclonal, ou fragment de celui-ci, selon l'une quelconque des revendications 6 ou 7.

9. Procédé selon l'une quelconque des revendications 1 à 5, comprenant la mise en contact de l'anticorps monoclonal ou fragment de celui-ci selon la revendication 6 ou 7 avec un échantillon, et la mesure de la quantité de RNase 1 pancréatique ayant formé un complexe avec ledit anticorps monoclonal ou fragment de celui-ci.

10. Procédé selon la revendication 9 ; comprenant en outre la mise en contact de l'anticorps monoclonal ou fragment de celui-ci selon la revendication 8 avec l'échantillon précédemment mis en contact avec l'anticorps monoclonal ou fragment de celui-ci selon la revendication 6 ou 7, et la mesure de la quantité de RNase 1 pancréatique ayant formé un complexe avec lesdits deux anticorps monoclonaux ou fragments de ceux-ci.

11. Procédé selon la revendication 9 ou 10, comprenant une première étape pour mettre en contact l'échantillon avec l'anticorps monoclonal ou fragment de celui-ci selon la revendication 6 ou 7 ou avec l'anticorps monoclonal ou fragment de celui-ci selon la revendication 8, et une seconde étape pour mettre en outre en contact l'échantillon obtenu à la première étape, respectivement, avec l'anticorps monoclonal ou fragment de celui-ci selon la revendication 8 ou avec l'anticorps monoclonal ou fragment de celui-ci selon la revendication 6 ou 7.

12. Composition pharmaceutique contenant l'anticorps monoclonal, ou fragment de celui-ci, selon l'une quelconque des revendications 6 ou 7.

13. Procédé selon l'une quelconque des revendications 1 à 5, dans lequel la quantité de site de N-glycosylation potentiel de la RNase 1 pancréatique lié à une chaine N-glycane ou non lié à une chaine N-glycane est déterminée par spectrométrie de masse.

14. Procédé selon la revendication 13, dans lequel la détermination par spectrométrie de masse inclut la décomposition de la RNase 1 pancréatique avec une enzyme et puis la mesure de la masse du fragment peptidique et/ou du

fragment glycopeptidique résultant.

# FIG. 1

# FIG. 2

# FIG. 3

Immunoprecipitation antibody:Anti-human kappa light chain antibody
Immunostaining antibody:Anti-RNase 1 antibody(RN15013)

# FIG. 4

FIG. 5

FIG. 6

$P = 7.3 \times 10^{-15}$

## FIG. 7

# FIG. 8

# FIG. 9

# FIG. 10

FIG. 11

# FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012131970 A

- JP 2012272285 A

**Non-patent literature cited in the description**

- *J. Clin. Pathol.,* 1980, vol. 33, 1212-13
- *Biol. Chem. Hoppe Seyler,* 1994, vol. 375, 357-63
- *Eur. J. Biochem.,* 2000, vol. 267, 1484-1494
- *Glycobiology,* 2003, vol. 13, 227-244
- *Glycobiology,* 2007, vol. 17, 388-400
- *Biochem. Biophys. Res.,* 1995, vol. 26 (1), 406-413
- *Cancer,* 2003, vol. 89 (6), 1252-1258
- *J. Proteome Res.,* 2004, vol. 3, 556-566